(19)

Europäisches Patentamt
European Patent Office
Office européen des brevets

(11)  **EP 3 412 308 A1**

(12)  **EUROPEAN PATENT APPLICATION**
published in accordance with Art. 153(4) EPC

(43) Date of publication:
**12.12.2018 Bulletin 2018/50**

(21) Application number: **17747468.1**

(22) Date of filing: **01.02.2017**

(51) Int Cl.:
*A61K 39/39* (2006.01)    *A61K 39/00* (2006.01)

(86) International application number:
**PCT/JP2017/003631**

(87) International publication number:
**WO 2017/135313 (10.08.2017 Gazette 2017/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:  **02.02.2016   JP 2016018278**

(71) Applicant: **Nitto Denko Corporation**
**Ibaraki-shi, Osaka 567-8680 (JP)**

(72) Inventors:
 • **ASARI, Daisuke**
   **Ibaraki-shi**
   **Osaka 567-8680 (JP)**

 • **SHISHIDO, Takuya**
   **Ibaraki-shi**
   **Osaka 567-8680 (JP)**
 • **MATSUSHITA, Kyohei**
   **Ibaraki-shi**
   **Osaka 567-8680 (JP)**
 • **HORI, Mitsuhiko**
   **Ibaraki-shi**
   **Osaka 567-8680 (JP)**

(74) Representative: **Müller-Boré & Partner**
**Patentanwälte PartG mbB**
**Friedenheimer Brücke 21**
**80639 München (DE)**

(54)  **COMPOSITION FOR IMMUNITY INDUCTION PROMOTION AND VACCINE PHARMACEUTICAL COMPOSITION**

(57)  The present invention aims to provide a composition for immunity induction promotion and a vaccine pharmaceutical composition each universally usable for induction of cellular immunity and/or humoral immunity against various antigens and exerting a high effect of inducing cellular immunity and/or humoral immunity. Provided is a composition for immunity induction promotion, containing a first immunity induction promoter, the first immunity induction promoter being an ion channel drug that affects an ion channel or an ion pump.

**EP 3 412 308 A1**

**Description**

TECHNICAL FIELD

The present invention relates to a composition for immunity induction promotion and a vaccine pharmaceutical composition. The present invention particularly relates to a composition for immunity induction promotion and a vaccine pharmaceutical composition each containing a medicament that affects an ion channel or an ion pump to control the intracellular ion concentration.

BACKGROUND ART

Common widely used vaccines are made from pathogens (e.g., microorganisms, viruses) or such pathogens whose toxicity is partially weakened or eliminated. The vaccines are administered to living bodies to induce immunity. Most of the vaccine preparations commercially available at the present are injections.

For induction of immunity by vaccine administration, injection such as subcutaneous injection, intradermal injection, or intramuscular injection is commonly employed. Vaccines have to be invasively administered into the body because microorganisms or viruses cannot enter the body through the skin due to their size.

Adjuvants commonly used in immunity induction by injection include aluminum salts (e.g., aluminum hydroxide, aluminum phosphate, aluminum chloride) and emulsions containing squalene (e.g., MF59, AS03). Other adjuvants, such as flagellar components, nucleic acids, cytokines, cationic polymers, and polypeptides are also being widely considered.

Injection, however, is disadvantageous in terms of the QOL of patients because it provides a physical and mental load such as pain, fear, and injection scars and subsequent cicatrization. In addition, when repetitive administrations are required, regular hospital visits put a load on patient life. Injection further has other disadvantages that injection is permitted only for healthcare workers, that intradermal injection, which has high immune effects, requires difficult manipulation, that healthcare workers have a risk of injection via needle pricking, and that injection generates medical wastes requiring special disposition, such as injection needles. Thus, injection is not necessarily the best administration route.

Since a large number of Langerhans cells that are antigen presenting cells are present in the skin, induction of humoral immunity by transdermal administration (see Patent Literature 1 and non-Patent Literature 1), buccal administration, transnasal administration, or sublingual administration (see Patent Literature 2, Patent Literature 3, and non-Patent Literature 2) is now considered as a means capable of solving the above various problems in relation to the injection.

As adjuvants for the route other than injection, such as induction of humoral immunity by transdermal administration or transmucosal administration, aluminum salts (e.g., aluminum hydroxide, aluminum phosphate, aluminum chloride) and toxins (e.g., cholera toxin, Escherichia coli heat-labile toxin) are being considered, but they are not yet in practical use. Moreover, almost no effective immunostimulants usable for induction of humoral immunity by transdermal administration of an antigen have been reported, and such immunostimulants often fail to exert a sufficient effect of inducing humoral immunity compared to the injection.

Immunity induction promoters which have been reported or brought into practical use include those contributing to extended release of antigens, those contributing to uptake of antigens into dendritic cells, and those contributing to the innate immune system such as Toll-like receptor ligands and NOD-like receptor ligands. They however are insufficient in the balance of safety and effect. Specifically, limited types of immunostimulants have been used to date, such as fragments derived from microorganisms or viruses (e.g., Toll-like receptor ligands), toxins such as cholera toxin and Escherichia coli heat-labile toxin, and oil-based adjuvants that utilize extended release of antigens to enhance the effect. They are insufficient in the balance of safety and effect.

The use of a Th2 cell differentiation inhibitor has been reported as a technique for effectively promoting cellular immunity (see Patent Literature 4). The Th2 cell differentiation inhibitor inhibits generation of prostaglandin E2 that promotes Th2 reaction, thereby relatively activating Th1 cells that activate cellular immunity. However, Patent Literature 4 only reports the evaluation on the technique for inducing cellular immunity, and the technique for effectively promoting humoral immunity that induces antibody production has not been reported.

CITATION LIST

- Patent Literature

Patent Literature 1: US 2008/0193487
Patent Literature 2: JP 2002-531415 T

Patent Literature 3: US 2008/0112974
Patent Literature 4: JP 2014-169281 A

- Non-Patent Literature

Non-Patent Literature 1: HOSOI AKIHIRO ET AL., CANCER RESEARCH, 68, 3941-3949 (2008)
Non- Patent Literature 2: ZHENGRONG CUI ET AL., PHARMACEUTICAL RESEARCH, VOL.19, NO.7, 947-953 (2002)

SUMMARY OF INVENTION

- Technical Problem

The present invention aims to provide a composition for immunity induction promotion and a vaccine pharmaceutical composition each universally usable for induction of cellular immunity and/or humoral immunity against various antigens and exerting a high effect of inducing cellular immunity and/or humoral immunity.

- Solution to problem

The present inventors made intensive studies to solve the above problems, and focused on the fact that control of the intracellular ion concentration of immunocompetent cells enables effective induction of cellular immunity characterized by cytotoxic T lymphocyte (CTL) production and/or humoral immunity characterized by antibody production.

Dendritic cells, a type of an immunocompetent cell engulfing viruses, microorganisms, or like foreign bodies migrate to lymph nodes and give naive T cells (Th0 cells) the information of the foreign bodies, thus inducing the differentiation of helper T cells. Through the interaction with dendritic cells, Th0 cells differentiate into type 1 helper T cells (Th1 cells), which are responsible for cellular immunity, and type 2 helper T cells (Th2 cells), which are responsible for humoral immunity. It is known that the direction of the differentiation of helper T cells is controllable with biological signaling molecules such as cytokines. Such control is widely employed in in vitro testing systems. The dendritic cells are activated by stimuli such as cytokines and assumed to determine the direction of the helper T cell differentiation through complicated signal transduction. That is, the "state" of dendritic cells decides the differentiation of T cells, leading to the induction of the cellular immunity or the humoral immunity. The former, where Th1 cells serve as the center of the reaction, is also called Th1 reaction. Similarly, the latter is also called Th2 reaction.

The present inventors focused on the above viewpoint and find out that the intracellular ion concentration is used as a signal transduction system when such immunocompetent cells are engulfing antigens. As a result of intensive study, the present inventors found out that the use of an ion channel drug that controls the intracellular ion concentration together with an antigen enables control of the immune route(s) of cellular immunity and/or humoral immunity. They found out that direct administration of an ion channel drug that controls the intracellular ion concentration together with an antigen to living bodies effectively induces antigen-specific cellular immunity and/or humoral immunity. Since ion channel drugs are widely used as medicaments, their safety to living bodies is ensured. They are also excellent in terms of the convenience. In addition, the present inventors found out that the use of a Toll-like receptor (TLR) ligand in combination further promotes cellular immunity and/or humoral immunity.

Specifically, the present invention relates to a composition for immunity induction promotion containing a first immunity induction promoter that is an ion channel drug that affects an ion channel or an ion pump.

The ion channel drug is preferably at least one selected from the group consisting of a sodium channel agonist, a calcium channel agonist, a potassium channel agonist, a chloride channel agonist, and a sodium/potassium pump agonist.

The present invention also relates to a vaccine pharmaceutical composition containing an antigen for immunity induction and the composition for immunity induction promotion.

The vaccine pharmaceutical composition preferably further contains a second immunity induction promoter that is an immunostimulant selected from the group consisting of a Toll-like receptor (TLR) ligand, a cyclic dinucleotide, and a helper peptide.

The vaccine pharmaceutical composition is preferably administered to a body surface.

The vaccine pharmaceutical composition is preferably administered by intradermal injection, subcutaneous injection, or intramuscular injection.

The present invention is specifically described in the following.

The composition for immunity induction promotion and vaccine pharmaceutical composition of the present invention are used for induction of humoral immunity and/or cellular immunity.

The humoral immunity inducing effect may be quantitatively determined by any method, and various methods have been developed. For example, the effect can be determined by an immunity induction test using an animal model for immunological evaluation and ELISA (antigen-specific IgG antibody). The sample for determining humoral immunity may be, for example, blood of the animal model for immunological evaluation.

The cellular immunity inducing effect may be quantitatively determined by any method, and various methods have been developed. For example, the effect can be determined by an immunity induction test using an animal model for immunological evaluation and ELISPOT (antigen-specific CTL number). The sample for determining cellular immunity may be, for example, spleen cells collected from the animal model for immunological evaluation.

The composition for immunity induction promotion of the present invention contains a first immunity induction promoter that is an ion channel drug that affects an ion channel or an ion pump (hereafter, also simply referred to as a first immunity induction promoter).

As used herein, the term "ion channel drug" means any substance that can affect an ion channel or an ion pump of an immunocompetent cell to improve the efficiency of inducing cellular immunity and/or humoral immunity to an antigen administered with the substance, as compared to the efficiency obtained without the substance. The substance is not limited by the mechanism of promoting cellular immunity and/or humoral immunity, but the term means those specified herein.

The ion channel drug that affects the ion channel is preferably at least one selected from the group consisting of a sodium channel agonist, a calcium channel agonist, a potassium channel agonist, and a chloride channel agonist.

As used herein, the term "sodium channel agonist" means a substance that affects the sodium channel to change the intracellular ion concentration. Sodium channel agonists are roughly classified into voltage-dependent sodium channel agonists and epithelial sodium channel agonists based on the mechanism.

Examples of the voltage-dependent sodium channel agonists include: lidocaine, mepivacaine, bupivacaine, levobupivacaine, ropivacaine, procaine, tetracaine, benzocaine, dibucaine, prilocaine, cocaine, mexiletine, flecainide, quinidine, carbamazepine, zonisamide, lamotrigine, ambroxol, and their derivatives; and pharmacologically acceptable salts of these.

Examples of the epithelial sodium channel agonists include: amiloride, triamterene, suramin, and their derivatives; and pharmacologically acceptable salts of these.

The sodium channel agonist is preferably amiloride, suramin, or triamterene.

As used herein, the term "calcium channel agonist" means a substance that affects the calcium channel to change the intracellular ion concentration. Calcium channel agonists are roughly classified into voltage-dependent calcium channel agonists and ion channel receptor calcium channel agonists based on the mechanism.

Examples of the voltage-dependent calcium channel agonists include: cilnidipine, ziconotide, dronedarone, amlodipine, felodipine, isradipine, nicardipine, nifedipine, nimodipine, nisoldipine, nitrendipine, lacidipine, nilvadipine, manidipine, barnidipine, benidipine, clevidipine, verapamil, gallopamil, diltiazem, fendiline, bepridil, perhexiline, topiramate, aranidipine, azelnidipine, darodipine, efonidipine, kirenidipine, mibefradil, ethosuximide, valproic acid, zonisamide, penfluridol, flunarizine, aranidipine, efonidipine, propitocaine, gabapentin, pregabalin, gabapentin enacarbil, lercanidipine, lidoflazine, metharbital, fluspirilene, belfosdil, brovincamine, clentiazem, flordipine, lomerizine, vatanidipine, and their derivatives; and pharmacologically acceptable salts of these.

Examples of the ion channel receptor calcium channel agonists include: topiramate, kainic acid, tezampanel, perampanel, farampator, tezampanel, talampanel, zonampanel, besonprodil, traxoprodil, ifenprodil, ketamine, esketamine, ketobemidone, flupirtine, memantine, methadone, acamprosate, dextromethorphan, aptiganel, delucemine, dextrorphan, dizocilpine, gavestinel, levomethadone, licostinel, latrepiridine, neramexane, perzinfotel, phencyclidine, remacemide, selfotel, tiletamine, and their derivatives; and pharmacologically acceptable salts of these.

The calcium channel agonist is preferably amlodipine or manidipine.

As used herein, the term "potassium channel agonist" means a substance that affects the potassium channel to change the intracellular ion concentration. Potassium channel agonists are roughly classified into voltage-dependent potassium channel agonists, calcium-dependent potassium channel agonists, inwardly rectifying potassium channel agonists, and tandem pore domain potassium channel agonists based on the mechanism.

Examples of the voltage-dependent potassium channel agonists include: pilsicainide, verapamil, propafenone, bepridil, clofilium, flecainide, amiodarone, tedisamil, dronedarone, nifekalant, linopirdine, azimilide, linopirdine, ezogabine, bretylium tosylate, aprindine, dofetilide, ibutilide, tedisamil, sotalol, cocaine, azimilide, clofilium, dexsotalol, nifekalant, sematilide, terikalant, tetraethylammonium, 4-aminopyridine, dendrotoxin, capsaicin, zucapsaicin, mavatrep, nonivamide, menthol, and their derivatives; and pharmacologically acceptable salts of these.

Examples of the calcium-dependent potassium channel agonists include: topiramate, kainic acid, tezampanel, perampanel, farampator, tezampanel, talampanel, zonampanel, besonprodil, traxoprodil, ifenprodil, ketamine, esketamine, etobemidone, flupirtine, memantine, methadone, acamprosate, dextromethorphan, aptiganel, delucemine, dextrorphan, dizocilpine, gavestinel, levomethadone, licostinel, latrepiridine, neramexane, peudinefotel, phencyclidine, remacemide, selfotel, tiletamine, and their derivatives; and pharmacologically acceptable salts of these.

Examples of the inwardly rectifying potassium channel agonists include: glibenclamide, tolbutamide, diazoxide, pinacidil, amiodarone, dronedarone, bepridil, tedisamil, nifekalant, terikalant, nicorandil, minoxidil, levcromakalim, and their derivatives; and pharmacologically acceptable salts of these.

Examples of the tandem pore domain potassium channel agonists include: halothane and its derivatives; and pharmacologically acceptable salts of these.

The potassium channel agonist is preferably dofetilide or glibenclamide.

As used herein, the term "chloride channel agonist" means a substance that affects the chloride channel to change the intracellular ion concentration. Chloride channel agonists are roughly classified into CIC chloride channel agonists, calcium-dependent chloride channel agonists, volume-regulated chloride channel agonists, and CFTR chloride channel agonists based on the mechanism.

Examples of the CIC chloride channel agonists include: lubiprostone and its derivatives; and pharmacologically acceptable salts of these.

Examples of the calcium-dependent chloride channel agonists include: niflumic acid and its derivatives; and pharmacologically acceptable salts of these.

The chloride channel agonist is preferably niflumic acid.

The ion channel drug that affect an ion pump are roughly classified into sodium/potassium pump agonists, proton/potassium pump agonists, calcium pump agonists, proton pump agonists, and copper ion pump agonists.

As used herein, the term "sodium/potassium pump agonist" means a substance that affects $Na^+/K^+$-ATPase (sodium/potassium pump) to change the intracellular ion concentration.

Examples of the sodium/potassium pump agonists include: digitoxin, digoxin, lanatoside C, deslanoside, methyl digoxin, acetyl digoxin, acetyl digitoxin, gitoformate, proscillaridin, G-strophanthin, and their derivatives; and pharmacologically acceptable salts of these.

The sodium/potassium pump agonist is preferably digitoxin or digoxin.

As used herein, the term "proton/potassium pump agonist" means a substance that affects $H^+/K^+$-ATPase (proton/potassium pump) to change the intracellular ion concentration.

Examples of the proton/potassium pump agonists include: omeprazole, pantoprazole, rabeprazole, esomeprazole, dexlansoprazole, lansoprazole, leminoprazole, picoprazole, tenatoprazole, timoprazole, and their derivatives; and pharmacologically acceptable salts of these.

As used herein, the term "salt" means any organic acid or inorganic acid, and preferably means a pharmacologically acceptable salt.

As used herein, the term "pharmacologically acceptable salt" means a salt that does not adversely affect the administration target and does not cause a loss of the pharmacological activity of the compounded materials in the vaccine pharmaceutical composition. Examples thereof include inorganic acid salts (e.g., hydrochloride, phosphate), organic acid salts (e.g., acetate, phthalate, and TFA salt), metal salts (e.g., alkali metal salts (e.g., sodium salt and potassium salt), alkaline earth metal salts (e.g., calcium salt and magnesium salt), and aluminum salts), and amine salts (e.g., triethylamine salt, benzylamine salt, diethanolamine salt, t-butylamine salt, dicyclohexylamine salt, arginine salt, dimethyl ammonium salt, and ammonium salt).

In the composition for immunity induction promotion of the present invention, the amount of the first immunity induction promoter is not particularly limited, and is preferably, based on the total mass of the composition for immunity induction promotion, 0.0001 to 100% by mass, more preferably 0.001 to 80% by mass, still more preferably 0.01 to 50% by mass.

If the amount of the first immunity induction promoter is less than 0.0001% by mass, the immunity inducing effect may be insufficient. If the amount of the first immunity induction promoter is more than 100% by mass, a safety issue may arise.

The composition for immunity induction promotion of the present invention may optionally contain additives.

Examples of the additives include a tonicity agent, an antiseptic preservative, an antioxidant, a solubilizer, a dissolution auxiliary, a suspending agent, a filler, a pH adjuster, a stabilizer, an absorption promoter, a release rate controlling agent, a colorant, a plasticizer, a crosslinking agent, and an adhesive. Each of these additives may be used alone or in combination of two or more thereof.

The vaccine pharmaceutical composition of the present invention contains an antigen and the composition for immunity induction promotion.

The vaccine pharmaceutical composition of the present invention containing the antigen and the composition for immunity induction promotion can effectively induce antigen-specific cellular immunity and/or humoral immunity.

As used herein, the term "antigen" means any substance capable of inducing an immune response. Any antigen may be used. Examples thereof include proteins and peptides. For transdermal administration which requires skin penetration of the antigen, antigens having small molecular weights are preferred. For example, a peptide having about 8 to 12 amino acid residues may be used.

The antigen is not particularly limited, and examples thereof include a cancer antigen peptide, an antigen derived from an infectious pathogen, and a human endogenous protein.

As used herein, the term "cancer" refers to the abnormal expression of oncogene. Examples of the cancer include a cancer associated with overexpression, such as a hematopoietic tumor or solid cancer.

As used herein, the term "abnormal expression of a gene" means that the expression level of a gene in a cell is significantly increased or decreased by, for example, at least two times or at least four times, as compared to another cell in the same tissue.

As used herein, the term "overexpression" means an abnormal increase in the expression level. The expression level of a gene can be easily measured by any method known in the relevant technical field.

Examples of the oncogene include survivin gene, GPC3 gene, HER2/neu gene, MAGE-3 gene, MAGE-Al gene, MAGE-A3/A6 gene, MAGE-A4 gene, MAGE-12 gene, proteinase-3 gene, AFP gene, CA-125 gene, CD44 gene, CEA gene, c-Kit gene, c-met gene, c-myc gene, L-myc gene, COX2 gene, CyclinDl gene, Cytokeratin-7 gene, Cytokeratin-19 gene, Cytokeratin-20 gene, E2F1 gene, E2F3 gene, EGFR gene, Gli1 gene, hCGβ gene, HIF-1α gene, HnRNP A2/B1 gene, hTERT gene, MDM gene, MDR-1 gene, MMP-2 gene, MMP-9 gene, Muc-1 gene, Muc-4 gene, Muc-7 gene, NSE gene, ProGRP gene, PSA gene, RCAS1 gene, SCC gene, thymoglobulin gene, VEGF-A gene, and VEGF-A gene.

Non-limiting examples of cancers associated with abnormal expression of the survivin gene include malignant lymphoma, bladder cancer, lung cancer, and large bowel cancer. Non-liming examples of cancers associated with abnormal expression of the GPC3 gene include liver cancer, bile duct cancer, and stomach cancer. Non-liming examples of cancers associated with abnormal expression of the HER2/neu gene include breast cancer, stomach cancer, ovarian cancer, uterine cancer, bladder cancer, non-small cell lung cancer, and prostatic cancer. Non-liming examples of cancers associated with abnormal expression of the MAGE-3 gene include melanoma, lung cancer, head and neck cancer, bladder cancer, stomach cancer, esophageal cancer, and liver cancer. Non-liming examples of cancers associated with abnormal expression of the proteinase-3 gene include acute myelocytic leukemia and pancreatic cancer.

As used herein, the term "cancer antigen" refers to a substance such as a protein or peptide which is specifically expressed in tumor cells or cancer cells and capable of inducing cellular immune response.

As used herein, the term "cancer antigen peptide" refers to a partial peptide derived from a cancer antigen protein, capable of inducing cellular immune response. Usually, a cancer antigen peptide is produced by decomposition of a cancer antigen protein (which is an oncogene product) in a cancer cell, and is presented on the surface of a cancer cell by MHC class I molecules.

The cancer antigen peptide may be an endogenous cancer antigen peptide isolated and purified from cancer cells, or may be a synthetic peptide having the same amino acid sequence as the endogenous cancer antigen peptide. Specifically, preferred examples of the cancer antigen peptide include survivin 2B peptide, GPC3 peptide, HER2/neu_A24 peptide, MAGE3_A24 peptide, IPEP87 peptide, PR1 peptide, HER2/neu_A02 peptide, MAGE3_A02 peptide, HBVenv peptide, HER2/neu_E75 peptide, MUC1 peptide, and altered peptides thereof.

As used herein, the term "survivin 2B peptide" refers to a peptide derived from survivin which is an oncogene product, having the sequence Ala Tyr Ala Cys Asn Thr Ser Thr Leu (SEQ ID No: 1).

As used herein, the term "GPC3 peptide" refers to a peptide derived from GPC3 which is an oncogene product, having the sequence Glu Tyr Ile Leu Ser Leu Glu Glu Leu (SEQ ID No: 2).

As used herein, the term "HER2/neu_A24 peptide" refers to an HLA-A24-restricted peptide derived from HER2/neu which is an oncogene product, having the sequence Thr Tyr Leu Pro Thr Asn Ala Ser Leu (SEQ ID No: 3).

As used herein, the term "MAGE3_A24 peptide" refers to an HLA-A24-restricted peptide derived from MAGE-3 which is an oncogene product, having the sequence Ile Met Pro Lys Ala Gly Leu Leu Ile (SEQ ID No: 4).

As used herein, the term "IPEP87 pepride" refers to a peptide derived from hepatitis C virus (HCV) protein, having the sequence Asp Leu Met Gly Tyr Ile Pro Ala Val (SEQ ID No: 5).

As used herein, the term "PR1 peptide" refers to a peptide derived from proteinase-3 which is an oncogene product, having the sequence Val Leu Gln Glu Leu Asn Val Thr Val (SEQ ID No: 6).

As used herein, the term "HER2/neu_A02 peptide" refers to an HLA-A02-restricted peptide derived from HER2/neu which is an oncogene product, having the sequence Lys Val Phe Gly Ser Leu Ala Phe Val (SEQ ID No: 7).

As used herein, the term "MAGE3_A02 peptide" refers to an HLA-A02-restricted peptide derived from MAGE-3 which is an oncogene product, having the sequence Lys Val Ala Glu Ile Val His Phe Leu (SEQ ID No: 8).

As used herein, the term "HBVenv peptide" refers to a peptide derived from hepatitis B virus (HBV) protein, having the sequence Trp Leu Ser Leu Leu Val Pro Phe Val (SEQ ID No: 9).

As used herein, the term "HER2/neu_E75 peptide" refers to a peptide derived from a product (HER2 protein) of an oncogene HER2/neu, having the sequence Lys Ile Phe Gly Ser Leu Ala Phe Leu (SEQ ID No: 10).

As used herein, the term "MUC1 peptide" refers to a peptide derived from MUC1 protein which is a glycoprotein that is highly expressed on many cancer cells, having the sequence Ser Thr Ala Pro Pro Val His Asn Val (SEQ ID No: 11) .

As used herein, the term "altered XX peptide" (XX represents any peptide name) refers to a XX peptide in which all or a part of amino acids are altered by, for example, substitution or modification.

Examples of the altered XX peptide include peptides such as: (a) a peptide having an amino acid sequence in which one to several amino acids (for example, 1, 2, 3, 4, 5 amino acids) are substituted, deleted, or added in the amino acid sequence of the XX peptide; and (b) a peptide having an amino acid sequence in which all or a part of amino acids (for example, one or multiple amino acids, e.g., 1, 2, 3, 4, 5, 6, 7, 8, 9, or 10 amino acids) are modified in the amino acid sequence of the XX peptide.

Non-limiting examples of the "modifications" of the amino acids in the altered XX peptide include acetylation; alkylation such as methylation; glycosylation; hydroxylation; carboxylation; aldehydation; phosphorylation; sulfonylation; formylation; aliphatic chain addition modification such as myristoylation, palmitoylation, and stearoylation; octanoylation; esterification; amidation; deamidation; disulfide bond formation modification such as cystine modification, glutathione modification, and thioglycolic acid modification; glycation; ubiquitination; succinimide formation; glutamylation; and prenylation. The altered XX peptide may contain substitution, deletion, or addition of one or more amino acids and modification of one or more amino acids in combination.

The peptides mentioned above can be in the free form or any pharmacologically acceptable salt form. Examples of the pharmacologically acceptable salt form include acid salts (e.g., acetate, TFA salt, hydrochloride, sulfate, phosphate, lactate, tartrate, maleate, fumarate, oxalate, hydrobromate, succinate, nitrate, malate, citrate, oleate, palmitate, propionate, formate, benzoate, picrate, benzenesulfonate, dodecylsulfate, methanesulfonate, p-toluenesulfonate, glutarate, and various amino acid salts), metal salts (e.g., alkali metal salts (e.g., sodium salt and potassium salt), alkaline-earth metal salts (e.g., calcium salt and magnesium salt), and aluminum salt), and amine salts (e.g., triethylamine salt, benzylamine salt, diethanolamine salt, t-butylamine salt, dicyclohexylamine salt, arginine salt, dimethylammonium salt, and ammonium salt). In particular, the pharmacologically acceptable salt is preferably acetate or TFA salt. The peptides mentioned above, which are usable as antigens in the present invention, can be synthesized or produced, isolated, and purified by a well-known method.

As used herein, "infectious disease pathogen-derived antigen" means an infectious disease pathogen or a component thereof or any substance derived therefrom, capable of inducing an immune response (e.g., maturation of an immunocompetent cell, increase in cytokine production, promotion of antibody production). An infectious disease can be treated or prevented by administering the infectious disease pathogen-derived antigen to a subject.

Examples of the proteins as antigens usable in the present invention include infectious pathogens, proteins derived from infectious pathogens, or human endogenous proteins.

The infectious diseases caused by the infectious pathogen are not limited. Examples thereof include virus diseases such as diseases caused by infection with adenovirus (e.g., human adenovirus), herpesvirus (e.g., herpes simplex virus, varicella-zoster virus, cytomegalovirus, human herpesvirus, Kaposi sarcoma-associated herpesvirus), picornavirus (e.g., poliovirus, common cold virus, hepatitis A virus), poxvirus (e.g., smallpox virus, vaccinia virus, molluscum contagiosum virus), picornavirus (e.g., rhinovirus, enterovirus), orthomyxovirus (e.g., influenza virus), paramyxovirus (e.g., parainfluenza virus, mumps virus, measles virus, respiratory syncytial virus (RSV), Newcastle disease virus), parvovirus (e.g., adeno associated virus), togavirus (e.g., rubella virus), coronavirus (e.g., SARS coronavirus), hepadnavirus (e.g., hepatitis B virus), flavivirus (e.g., Japanese encephalitis virus, yellow fever virus, dengue virus, West Nile fever virus, St. Louis encephalitis virus, Murray Valley encephalitis virus, hepatitis C virus, hepatitis G virus), hepevirus (e.g., hepatitis E virus), papillomavirus (e.g., human papilloma virus), calicivirus (e.g., norovirus), rhabdovirus (e.g., rabies virus, vesicular stomatitis virus), filovirus (e.g., Ebola hemorrhagic fever virus), arenavirus (e.g., Lassa virus, hepatitis D virus), bunyavirus (e.g., California encephalitis virus, Rift Valley fever virus), reovirus (e.g., rotavirus), or retrovirus (e.g., human immunodeficiency virus (HIV), adult T-cell leukemia virus); bacterial diseases such as those caused by infection with a bacterium such as Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococci, Neisseria, Clostridium, Bacillus, Corynebacterium, Mycobacterium, Campyrobacter, Vibrio, Serratia, Providencia, Chromobacterium, Brucella, Yersinia, Haemophilus, or Bordetella; fungous diseases such as chlamydia, candidiasis, aspergillosis, histoplasmosis, and cryptococcal meningitis; malaria; pneumocystis carinii pneumonia; leishmaniasis; cryptosporidiosis; toxoplasmosis; and Trypanosoma infection.

The human endogenous proteins usable in the present invention are not limited, and examples thereof include survivin 2B, GPC3, HER2/neu, MAGE3, IPEP87, PR1, HER2/neu E75, and MUC1.

The amount of the antigen in the vaccine pharmaceutical composition of the present invention is not limited. The amount of the antigen based on the total mass of the vaccine pharmaceutical composition is preferably 0.000001 to 50% by mass, more preferably 0.00001 to 20% by mass.

If the amount of the antigen based on the total mass of the vaccine pharmaceutical composition is less than 0.000001% by mass, the infection control or the effect as a therapeutic agent may be insufficient. If the amount is more than 50% by mass, a safety issue may arise.

The amount of the first immunity induction promoter in the vaccine pharmaceutical composition of the present invention is not limited. The amount of the first immunity induction promoter per 1 part by mass of the antigen is preferably 0.001 to 10,000 parts by mass, more preferably 0.01 to 10,000 parts by mass.

If the amount of the first immunity induction promoter per 1 part by mass of the antigen is less than 0.001 parts by mass, the immunity induction effect may be insufficient. If the amount of the first immunity induction promoter per 1 part by mass of the antigen is more than 10,000 parts by mass, a safety issue may arise.

The vaccine pharmaceutical composition of the present invention preferably further contains a second immunity induction promoter that is at least one immunostimulant selected from the group consisting of a Toll-like receptor (TLR) ligand, a cyclic dinucleotide, and a helper peptide (hereafter, also simply referred to as a second immunity induction promoter).

The use of the second immunity induction promoter in combination further promotes cellular immunity and/or humoral immunity.

The Toll-like receptor (TLR) ligand is a family of type I transmembrane proteins. The Toll-like receptor initiates, by in vivo activation thereof, an innate immune response in which a specific cytokine, a specific chemokine, and a specific growth factor are involved. While all the TLR's can activate certain intracellular signaling molecules (e.g., nuclear factor $\kappa$B (NF-$\kappa$B), mitogen-activated protein kinase (MAP kinase), a specific set of released cytokines and chemokines seems to be inherent to each TLR. TLR3, TLR7, TLR8, and TLR9 include a subfamily of TLR which is present in an endosome fraction or a lysosome fraction of an immune cell (e.g., dendritic cell and monocyte). Specifically, TLR3 is expressed by a wide range of cells such as dendritic cells and fibroblasts. TLR7 is expressed by plasma cell-like dendritic cells and is also expressed by monocytes to a lesser extent. TLR8 is expressed by monocytes, monocyte-derived dendritic cells, and myeloid dendritic cells. TLR9 is expressed by plasma cell-like dendritic cells. This subfamily mediates recognition of a microorganisms nucleic acid (single-stranded RNA, double-stranded RNA, single-stranded DNA, or the like). TLR3, TLR7 and/or TLR8, and TLR9 agonists stimulate production of various inflammatory cytokines

(e.g., interleukin-6, interleukin-12, TNF-$\alpha$, and interferon-$\gamma$). Such agonists also promote an increase in expression of costimulatory molecules (e.g., CD40, CD80, and CD86), major histocompatibility complex molecules, and chemokine receptors. Type I interferons (IFN$\alpha$ and IFN$\beta$) are also produced by cells upon activation with TLR7 and/or TLR8 agonist(s).

The cyclic dinucleotide is preferably a cyclic dipurine nucleotide, and may be a derivative or salt thereof as long as it holds the characteristic features. In terms of the safety, it is preferably, for example, c-di-GMP that is a cyclic bisguanosine monophosphate, c-di-AMP that is a cyclic bisadenosine monophosphate, or a derivative or salt thereof.

In the case where a helper peptide is used as the second immunity induction promoter, it is more preferably used as an immunostimulant for induction of cellular immunity.

As used herein, the term "helper peptide" refers to any peptide that activates helper-T cells.

Examples of the second cellular immunity induction promoter that is a helper peptide include a helper peptide derived from tubercle bacillus, a helper peptide derived from measles virus, a helper peptide derived from hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, a helper peptide derived from tetanus toxin, a helper peptide derived from pertussis toxin, a helper peptide derived from diphtheria toxin, helper peptides derived from cancer cells (e.g., IMA-MMP-001 helper peptide, CEA-006 helper peptide, MMP-001 helper peptide, TGFBI-004 helper peptide, HER-2/neu(aa776-790) helper peptide, AE36 helper peptide, AE37 helper peptide, MET-005 helper peptide, and BIR-002 helper peptide), and universal helper analogs (e.g., PADRE), and altered peptides thereof. Preferred among these are Peptide-25, altered Peptide-25, and PADRE.

As used herein, the term "PADRE" refers to a 13-amino acid peptide having the sequence D-Ala Lys cyclohexyl-Ala Val Ala Ala Trp Thr Leu Lys Ala Ala D-Ala (SEQ ID No: 12).

The amount of the second immunity induction promoter in the composition for immunity induction promotion and the vaccine pharmaceutical composition of the present invention is not limited. The amount of the second immunity induction promoter per 1 part by mass of the antigen is preferably 0.001 to 500 parts by mass, more preferably 0.005 to 200 parts by mass, still more preferably 0.01 to 100 parts by mass.

If the amount of the second immunity induction promoter is smaller than the lower limit, the immunity inducing effect may be insufficient. If the amount of the second immunity induction promoter is larger than the upper limit, a safety issue may arise.

The vaccine pharmaceutical composition of the present invention may be administered intradermally, subcutaneously, or intramuscularly but is preferably administered to a body surface, more preferably administered transdermally or transmucosally. In other words, the vaccine pharmaceutical composition of the present invention may be a vaccine pharmaceutical composition for intradermal, subcutaneous, or intramuscular administration, but is preferably a vaccine pharmaceutical composition for transdermal administration or transmucosal administration. Transdermal administration or transmucosal administration of the vaccine pharmaceutical composition of the present invention to the subject can effectively induce an antigen-specific cellular immunity and/or humoral immunity.

As used herein, the term "subject" refers to any animal in which administration of the vaccine pharmaceutical composition in practical use may induce an immune response. Typically, the term refers to mammals including human, mouse, rat, canine, feline, leporine, equine, bovine, ovine, porcine, caprine, simian, and chimpanzee. A particularly preferred subject is a human.

<Vaccine pharmaceutical composition for transmucosal administration>

The vaccine pharmaceutical composition for transmucosal administration of the present invention exhibits high humoral immunity inducing effect in transmucosal administration of various antigens to the target.

Examples of the transmucosal administration include administration to the tongue (e.g., under the tongue, back of the tongue), transnasal administration, buccal administration, rectal administration, and vaginal administration.

The dosage form of the vaccine pharmaceutical composition for transmucosal administration may be, for example, a semisolid formulation such as a gel (jelly), a cream, an ointment, and a plaster; a solution; a solid formulation such as a powder, a fine granule, a granule, a film, a tablet, and an orally disintegrating tablet; a mucosal spray formulation such as an aerosol; or an inhalant. Categories, definitions, properties, production processes, and the like of these formulations are well known in the relevant art. For example, see the Japanese Pharmacopoeia, 16th Edition.

Examples of the solvent used for the solution include an appropriate amount of water, ethanol, glycerin, or propylene glycol. The solution can be prepared by dispersing or dissolving the components in the solvent.

Examples of a base used for the gel (jelly) include hydrogel bases such as a carboxy vinyl polymer, a gel base, a nonfat ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, a carboxy vinyl polymer, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, eudragit, casein, alkyl alginate ester, gelatin, and polyethylene glycol. A fluidic gel or a formable gel can be prepared by dissolving any of these bases in a solvent and adding the above ingredients thereto. The solvent is preferably water, but glycerin or propylene glycol may also be used.

Examples of a base used for the cream include water/oil-type bases such as hydrophilic ointment and vanishing cream; and oil/water-type bases such as hydrophilic petrolatum, purified lanolin, Aquahole, Eucerin, Neocerin, hydrous lanolin, cold cream, and hydrophilic plastibase. Any of these bases is placed in an oil/fat solvent or water and then stirred at high speed using a homogenizer or the like, whereby a cream can be prepared.

Examples of a base used for the film include polyvinylpyrrolidone, polyvinyl alcohol, sodium polyacrylate, carboxymethylcellulose, starch, xanthan gum, karaya gum, sodium alginate, methylcellulose, carboxyvinyl polymer, agar, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymer, tragacanth, gum arabic, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl aminoacetate, casein, and alkyl alginate ester. Any of these bases is dissolved in water or a polar organic solvent (e.g., ethanol), blended with the above ingredients, applied to form a thin film, and then dried, whereby a film can be prepared.

Examples of additives for the powder, fine granule, granule, and tablets include excipients such as lactose, corn starch, and crystalline cellulose, and binders such as hydroxypropyl cellulose and gum arabic. Any of these additives is added to an appropriate amount of solvent such as water or ethanol, and the above ingredients are added thereto. They are mixed and kneaded, and then subjected to a combination of processes such as granulation, drying, and tableting, whereby a powder, fine granule, granule, or tablet can be prepared. If necessary, a lubricant such as magnesium stearate or a coating agent such as hydroxypropyl cellulose or sucrose can also be added.

Examples of a base usable for the orally disintegrating tablet (freeze dry type) include polysaccharides such as gelatin and pullulan. In addition, a forming aid such as mannitol, trehalose, sorbitol, or glycine may be used. The orally disintegrating tablet (freeze dry type) can be prepared by dissolving any of these bases and a forming aid in water, adding the above ingredients, dispensing the solution, and freeze-drying the solution.

Examples of the contents of the aerosol include a solution, a gel having high fluidity, cream, fine powder such as a powdered drug, or the like. The aerosol can be efficiently administered to a site of administration, for example, oral mucosa or nasal mucosa, by dispersing the solid or liquid fine particles in a gas with an atomizing device.

The vaccine pharmaceutical composition for transmucosal administration of the present invention is preferably a film or an orally disintegrating tablet.

<Vaccine pharmaceutical composition for intradermal, subcutaneous, or intramuscular administration>

The vaccine pharmaceutical composition for intradermal, subcutaneous, or intramuscular administration of the present invention exhibits high cellular immunity inducing effect in intradermal, subcutaneous, or intramuscular administration of various antigens to the target.

The dosage form of the vaccine pharmaceutical composition for intradermal, subcutaneous, or intramuscular administration is an injectable form with a certain level of fluidity, and may be, for example a solution, a watersoluble or hydrophobic suspension, or a cream. Categories, definitions, properties, production processes, and the like of these formulations are well known in the relevant art. For example, see the Japanese Pharmacopoeia, 16th Edition.

A solvent used for the solution may be an appropriate amount of water, saline, ethanol, glycerin, or propylene glycol. A solution can be prepared by dispersing or dissolving the above ingredients in any of these solvents.

Examples of a base usable for the aqueous suspension include hydrogel bases such as carboxyvinyl polymers, gel bases, nonfat ointment, polyvinylpyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymers, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, Eudragit, casein, alkyl alginate ester, gelatin, and polyethylene glycol. A fluidic suspension can be prepared by dissolving any of these bases into a solvent and adding the above ingredients thereto. The solvent is preferably saline, but glycerin or propylene glycol can also be used.

Examples of a base for hydrophobic suspensions include water/oil-type bases such as hydrophilic ointment and vanishing cream; and oil/water-type bases such as hydrophilic petrolatum, purified lanolin, Aquahole, Eucerin, Neocerin, hydrous lanolin, cold cream, and hydrophilic plastibase. A hydrophobic suspension can be prepared by placing any of these bases into a fat/oil solvent or water, stirring the mixture at a high speed with a homogenizer or the like, and adding the above ingredients thereto.

<Vaccine pharmaceutical composition for transdermal administration>

The dosage form of the vaccine pharmaceutical composition for transdermal administration may be, for example, a solution for external application such as a liniment or a lotion; a spray for external application such as an aerosol; a gel; a patch such as a tape or a poultice; an ointment; a plaster; or a cream. Categories, definitions, properties, production processes, and the like of these formulations are well known in the relevant art. For example, see the Japanese Pharmacopoeia, 16th Edition.

The amounts of the antigen and the ion channel drug in the vaccine pharmaceutical composition for transdermal administration are not limited. The amount of the antigen is preferably 0.01 to 40% by mass, more preferably 0.1 to 30% by mass. The amount of the ion channel drug is preferably 0.001 to 30% by mass, more preferably 0.01 to 20% by mass.

Examples of a base usable for the liniment include water, ethanol, fatty oils, hard paraffin, soft paraffin, liquid paraffin, glycerin, paraffin oil, beeswax, metal soap, mucilage, natural oils (such as almond oil, corn oil, peanut oil, castor oil, olive oil and derivatives thereof (e.g., polyoxyl castor oil)), mutton tallow or derivatives thereof, and fatty acids and/or esters thereof (e.g., stearic acid, oleic acid, isopropyl myristate).

The lotion is a formulation containing materials (i.e., the antigen and the ion channel drug) finely and homogeneously dispersed in an aqueous solution, and may be a suspension-type lotion and an emulsion-type lotion. Examples of the suspending agent include gum arabic, sodium alginate, sodium carboxymethylcellulose, methylcellulose, and bentonite. Examples of the emulsifier include sodium lauryl sulfate and sorbitan fatty acid esters.

Examples of a base usable for the ointment include those commonly used as hydrophobic bases such as oils/fats, waxes, and hydrocarbon compounds. Specific examples thereof include mineral bases such as yellow petrolatum, white petrolatum, paraffin, liquid paraffin, plastibase, and silicone, and animal or plant bases such as beeswax and animal or plant fats and/or oils.

Examples of a base usable for the cream include water/oil-type bases such as hydrophilic ointment and vanishing cream; and oil/water-type bases such as hydrophilic petrolatum, purified lanolin, Aquahole, Eucerin, Neocerin, hydrous lanolin, cold cream, and hydrophilic plastibase.

Examples of a base usable for the gel include hydrogel bases such as carboxyvinyl polymers, gel bases, nonfat ointment, polyvinyl pyrrolidone, polyvinyl alcohol, sodium polyacrylate, carboxymethylcellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxyvinyl polymers, tragacanth, gum arabic, tara gum, tamarind seed gum, psyllium seed gum, agar, gellan gum, glucomannan, locust bean gum, guar gum, carrageenan, dextrin, dextran, amylose, potassium carboxymethylcellulose, sodium carboxymethylcellulose, calcium carboxymethylcellulose, pullulan, chitosan, sodium carboxymethyl starch, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl aminoacetate, casein, alkyl alginate ester, gelatin, and polyethylene glycol.

Examples of a base usable for the poultice include gelatin, sodium carboxymethylcellulose, methylcellulose, sodium polyacrylate, kaolin, polyvinyl alcohol, polyvinyl pyrrolidone, glycerin, propylene glycol, and water.

The tape preferably includes an adhesive layer formed from the vaccine pharmaceutical composition containing materials (i.e., the antigen, the ion channel drug) and a support that supports the adhesive layer. The tape may further include a release liner that prevents exposure of the adhesive layer before use and that can be easily removed from the adhesive layer at the time of use.

Any adhesive may be used to form the adhesive layer. Examples of the adhesive include: acrylic adhesives containing acrylic polymers; rubber adhesives containing rubber elastomers such as synthetic isoprene rubber, polyisobutylene (PIB), butyl rubber, polybutadiene, styrene-butadiene rubber, and styrene-isoprene-styrene (SIS) rubber; styrene-diene-styrene block copolymers such as styrene-isoprene-styrene block copolymers, and styrene-butadiene-styrene block copolymers; silicone adhesives such as silicone rubber, dimethyl siloxane adhesives, and diphenyl siloxane adhesives; vinyl ether adhesives such as polyvinyl methyl ether, polyvinyl ethyl ether, and polyvinyl isobutyl ether; vinyl ester adhesives such as vinyl acetate-ethylene copolymers; polyester adhesives containing a carboxylic acid component (e.g., dimethyl terephthalate, dimethyl isophthalate, dimethyl phthalate) and a polyhydric alcohol component (e.g., ethylene glycol). Particularly preferred adhesives are acrylic adhesives, rubber adhesives, and silicone adhesives. The amount of the adhesive in the adhesive layer is not limited. The amount of the adhesive as the solid content is preferably 10 to 90% by mass, more preferably 20 to 80% by mass of the total mass of the adhesive layer.

The amounts of the antigen and the ion channel drug in the adhesive layer are not limited. The amount of the antigen is preferably 0.01 to 40% by mass, more preferably 0.1 to 30% by mass. The amount of the ion channel drug is preferably 0.001 to 30% by mass, more preferably 0.01 to 20% by mass.

The acrylic adhesive preferably contains, as a main component, a polymer that contains a C2-C18 alkyl (meth)acrylate as a first monomer.

Examples of the first monomer include alkyl (meth)acrylates containing a C1-C18 linear, branched, or cyclic alkyl group (e.g., methyl, ethyl, propyl, butyl, pentyl, hexyl, cyclohexyl, heptyl, octyl, 2-ethylhexyl, nonyl, decyl, undecyl, dodecyl, and tridecyl). Preferred among these are alkyl (meth)acrylates containing a C4-C18 linear, branched, or cyclic alkyl group (e.g., butyl, pentyl, hexyl, cyclohexyl, heptyl, octyl, 2-ethylhexyl, nonyl, decyl, undecyl, dodecyl, and tridecyl). Further, since a monomer component that lowers the glass transition temperature of a polymer is suitably used to impart adhesiveness at room temperature, an alkyl (meth)acrylate containing a C4-C8 linear, branched, or cyclic alkyl group (e.g., butyl, pentyl, hexyl, cyclohexyl, heptyl, octyl, or 2-ethylhexyl; preferably butyl, 2-ethylhexyl, or cyclohexyl; particularly preferably 2-ethylhexyl) is more preferred.

Specifically, the first monomer is preferably butyl acrylate, 2-ethylhexyl acrylate, 2-ethylhexyl methacrylate, cyclohexyl acrylate, or cyclohexyl methacrylate, particularly preferably 2-ethylhexyl acrylate. These first monomers may be used alone or in combination of two or more thereof.

The first monomer may be copolymerized with a second monomer, and examples of such a second monomer include monomers having a functional group that can be a crosslinking point when a crosslinking agent is used. Examples of such a functional group capable of being involved in crosslinking reaction include a hydroxyl group, a carboxyl group, and a vinyl group, with a hydroxyl group and a carboxyl group being preferred.

Examples of the second monomer include hydroxy ethyl (meth)acrylate, hydroxypropyl (meth)acrylate, N-hydroxyalkyl (meth)acrylamide, (meth)acrylic acid, itaconic acid, maleic acid, maleic anhydride, mesaconic acid, citraconic acid, and glutaconic acid. From the viewpoint of availability, acrylic acid, methacrylic acid, hydroxyethyl acrylate (in particular, 2-hydroxyethyl acrylate) are preferred, with acrylic acid being particularly preferred. These second monomers can be used alone or in combination of two or more thereof.

The first monomer and second monomer may be further copolymerized with a third monomer. Examples of the third monomer include vinyl esters such as vinyl acetate and vinyl propionate; vinyl ethers such as methyl vinyl ether and ethyl vinyl ether; vinyl amides such as N-vinyl-2-pyrrolidone and N-vinylcaprolactam; alkoxy (meth)acrylates such as methoxyethyl (meth)acrylate, ethoxyethyl (meth)acrylate, and tetrahydrofurfuryl (meth)acrylate; hydroxyl group-containing monomers (as the third monomer, not as a crosslinking point) such as hydroxypropyl(meth)acrylate and α-hydroxymethyl acrylate; (meth)acrylic acid derivatives having an amide group such as (meth)acrylamide, dimethyl (meth)acrylamide, N-butyl (meth)acrylamide, and N-methylol (meth)acrylamide; aminoalkyl (meth)acrylates such as aminoethyl (meth)acrylate, dimethylaminoethyl (meth)acrylate, and t-butyl aminoethyl (meth)acrylate; alkoxyalkylene glycol (meth)acrylates such as methoxyethylene glycol (meth)acrylate, methoxydiethylene glycol (meth)acrylate, methoxypolyethylene glycol (meth)acrylate, and methoxypolypropylene glycol (meth)acrylate; (meth)acrylonitrile; monomers containing sulfonic acid such as styrenesulfonic acid, allylsulfonic acid, sulfopropyl(meth)acrylate, (meth)acryloyloxy naphthalene sulfonate, and acrylamide methylsulfonate; and vinyl group-containing monomers such as vinylpiperidone, vinylpyrimidine, vinylpiperazine, vinylpyrrole, vinylimidazole, vinyloxazole, and vinylmorpholine. Preferred among these are vinyl esters and vinyl amides. Vinyl acetate is preferred among vinyl esters, and N-vinyl-2-pyrrolidone is preferred among vinyl amides. These third monomers may be used alone or in combination of two or more thereof.

In the case of a copolymer of the alkyl (meth)acrylate (first monomer) and the vinyl monomer having a functional group capable of being involved in crosslinking reaction (second monomer), the alkyl (meth)acrylate and the vinyl monomer having a functional group capable of being involved in crosslinking reaction are preferably copolymerized at a weight ratio of (99-85): (1-15), more preferably at a weight ratio of (99-90) : (1-10). In the case of a copolymer of the alkyl (meth)acrylate (first monomer), the vinyl monomer having a functional group capable of being involved in crosslinking reaction (second monomer), and a different monomer other than these (third monomer), the alkyl (meth)acrylate, the vinyl monomer having a functional group capable of being involved in crosslinking reaction, and the different monomer are preferably copolymerized at a weight ratio of (40-94):(1-15):(5-50), more preferably at a weight ratio of (50-89): (1-10) : (10-40) .

The polymerization reaction may be carried out by any conventionally known method. For example, the above monomers may be reacted in the presence of an initiator (e.g., benzoyl peroxide or azobisisobutyronitrile) in a solvent (e.g., ethyl acetate) at 50°C to 70°C for 5 to 48 hours.

The acrylic adhesive more preferably contains a 2-ethylhexyl acrylate/acrylic acid/N-vinyl-2-pyrrolidone copolymer, a 2-ethylhexyl acrylate/N-(2-hydroxyethyl)acrylamide/N-vinyl-2-pyrrolidone copolymer, a 2-ethylhexyl acrylate/2-hydroxyethyl acrylate/vinyl acetate copolymer, or a 2-ethylhexyl acrylate/acrylic acid copolymer, particularly preferably contains a 2-ethylhexyl acrylate/acrylic acid/N-vinyl-2-pyrrolidone copolymer.

The acrylic adhesive may be subjected to physical crosslinking treatment by radiation such as ultraviolet irradiation or electron beam irradiation, or chemical crosslinking treatment using various crosslinking agents such as an isocyanate compound (e.g., trifunctional isocyanate), an organic peroxide, an organic metal salt, a metal alcoholate, a metal chelate compound, or a polyfunctional compound (e.g., a polyfunctional external crosslinking agent, a polyfunctional monomer for internal crosslinking such as di(meth)acrylate).

Examples of rubber elastomers for preparing the rubber adhesive include polyisobutylene/polybutene elastomer, styrene/diene/styrene block copolymer, styrene/butadiene elastomer, nitrile elastomer, chloroprene elastomer, vinylpyridine elastomer, polyisobutylene elastomer, butyl elastomer, and isoprene-isobutylene elastomer. Preferred among these are polyisobutylene (PIB) and styrene/diene/styrene block copolymer (such as styrene/butadiene/styrene block copolymer (SBS) or styrene/isoprene/styrene block copolymer (SIS)), in view of solubility to the materials and the skin adhesiveness. These rubber elastomers may be used alone or in combination of two or more thereof.

In order to achieve appropriate adhesion and solubility to the materials, the rubber adhesive may be a mixture of rubber elastomers prepared from the same or different components and different in the average molecular weight. For example, a mixture of a high-molecular-weight polyisobutylene having an average molecular weight of 150,000 to 5,500,000 and a medium-molecular-weight polyisobutylene having an average molecular weight of 10,000 to 150,000, and/or a low-molecular-weight polyisobutylene having an average molecular weight of 500 to 4,000 is preferred. The amount of the high-molecular-weight polyisobutylene is 10 to 80% by mass, preferably 20 to 70% by mass, relative to the total amount of the polyisobutylenes. The amount of the medium-molecular-weight polyisobutylene is 0 to 90% by mass, preferably 10 to 80% by mass, relative to the total amount of the polyisobutylenes. The amount of the low-molecular-weight polyisobutylene is 0 to 80% by mass, preferably 10 to 60% by mass, relative to the total amount of the polyisobutylenes.

As used herein, the term "average molecular weight" refers to a viscosity average molecular weight calculated from the Flory viscosity equation. The average molecular weight is determined by calculating the Staudinger index ($J_0$) from the flow time at 20°C of the capillary 1 of an Ubbelohde viscometer by the Schulz-Blaschke equation, and using this $J_0$ value in the following expression.

$$J_0 = \eta_{sp} / c \, (1 + 0.31 \eta_{sp}) \quad \text{(Shults-Blaschke equation)}$$

$$\eta_{sp} = t / t_0 - 1$$

t: Flow time of solution (according to Hagenbach-couette correction formula)
$t_0$: Flow time of solvent (according to Hagenbach-couette correction formula)
c: Concentration of solution ($g/cm^3$)

$$J_0 = 3.06 \times 10^{-2} \overline{M}v^{0.65}$$

$\overline{\text{Mv}}$: Viscosity average molecular weight

In order to achieve appropriate tackiness, the rubber adhesive may contain a tackifier such as rosin resin, polyterpene resin, coumarone-indene resin, petroleum resin, terpene-phenol resin, xylene resin, or alicyclic saturated hydrocarbon resin. These tackifiers may be used alone or in combination of two or more thereof.

The amount of the tackifier is preferably 50% by mass or less, more preferably 5 to 40% by mass relative to the total mass of the rubber adhesive.

Examples of the silicone adhesive include polyorganosiloxane adhesives, polydimethylsiloxane adhesives, and polydimethyldiphenyl-siloxane adhesives. In particular, commercially available silicone adhesives such as BIO PSA (Dow Corning Corporation) are preferred.

The adhesive layer may further contain a skin permeation enhancer.

As used herein, the term "skin permeation enhancer" means any substance that may improve the efficiency of skin permeation of a transdermally administered antigen.

The skin permeation enhancer is preferably liquid (i.e., having fluidity) at room temperature (25°C). In the case where two or more types of skin permeation enhancers are mixed, the final mixture is preferably liquid at room temperature (25°C) and has an effect of enhancing skin permeation. Such an organic liquid component is preferably a hydrophobic liquid component in terms of the compatibility in the adhesive.

Examples of the skin permeation enhancer include higher alcohols, fatty acid esters, and polyhydric alcohol fatty acid esters.

The higher alcohol is preferably a C8-C18 higher alcohol, more preferably a C8-C14 higher alcohol. The fatty acid ester is preferably a fatty acid ester of a C8-C18 fatty acid and a C1-C18 monohydric alcohol, more preferably a fatty acid ester of a C12-C16 fatty acid and a C1-C18 monohydric alcohol. In particular, preferred are fatty acid esters, and particularly preferred are isopropyl myristate, isopropyl palmitate, and diethyl sebacate.

The amount of the skin permeation enhancer based on the total mass of the adhesive layer is preferably 0.1% by mass to 70% by mass, more preferably 1% by mass to 65% by mass, still more preferably 5% by mass to 60% by mass. When the proportion of the skin permeation enhancer is 0.1% by mass or more, the effect of promoting transdermal absorption obtained is high. When the proportion of the skin permeation enhancer is 70% by mass or less, high transdermal absorption is advantageously achieved while reduction in the adhesion force or cohesion force of the entire adhesive layer is suppressed.

Examples of the skin permeation enhancer include higher alcohols such as oleyl alcohol and octyldodecanol; polyhydric alcohols such as glycerin, ethylene glycol, and polypropylene glycol; higher fatty acids such as oleic acid and caprylic acid; fatty acid esters such as isopropyl myristate, isopropyl palmitate, and ethyl oleate; polybasic acid esters such as diethyl sebacate and diisopropyl adipate; polyhydric alcohol fatty acid esters such as diglyceryl triisostearate, sorbitan monooleate, propylene glycol dicaprylate, polyethylene glycol monolaurate, and polyoxyethylene sorbitol tetraoleate; polyoxyethylene alkyl ethers such as polyoxyethylene lauryl ether; hydrocarbons such as squalane and liquid paraffin; vegetable oils such as olive oil and castor oil; silicone oil; pyrrolidones such as N-methylpyrrolidone and N-dodecyl pyrrolidone; and sulfoxides such as decyl methyl sulfoxide. These skin permeation enhancers may be used alone or in combination of two or more thereof.

In the case of using the acrylic adhesive or rubber adhesive, the skin permeation enhancer used may be, for example, polyvinyl pyrrolidone, crospovidone, polypropylene glycol, polyvinyl alcohol, carboxy vinyl polymer, hydroxypropyl cellulose, or a mixture of these. Preferred among these are polyvinyl pyrrolidone, crospovidone, and polypropylene glycol.

The adhesive layer may have any thickness. Preferably, the thickness is 10 to 1000 μm. With the thickness within the above range, the adhesive layer can readily contain the materials each in an effective amount and exhibit sufficient adhesion. Moreover, the adhesive layer with such a thickness can be readily formed.

The support is not particularly limited, and is preferably one substantially impermeable to the above materials. In other words, it is preferably one that prevents a decrease in the amounts of the antigen, the ion channel drug, and, if contained, the adjuvant in the adhesive layer by not allowing them to pass through the support and escape from the back side.

The support may be a single film of polyester, polyamide, polyvinylidene chloride, polyethylene, polypropylene, polyvinyl chloride, ethylene-ethyl acrylate copolymer, polytetrafluoroethylene, ionomer resin, metal foil, or the like, or it may be a laminated film of these mentioned above. Preferred among these is a laminated film of a nonporous plastic film which is made of any of the above-mentioned materials and a porous film, in view of achieving good adhesiveness (anchoring properties) between the support and the adhesive layer. In this case, the adhesive layer is preferably formed on the porous film side.

Any porous film that improves the anchoring properties between the support and the adhesive layer may be used. Examples thereof include paper, woven fabrics, nonwoven fabrics, knitted fabrics, and mechanically perforated sheets. Preferred among these are paper, woven fabrics, and nonwoven fabrics, in view of factors such as handleability.

A porous film having a thickness in the range of 1 to 200 μm is preferred in view of improving anchoring properties and also in view of factors such as flexibility and attachment operability of the tape. In addition, in the case where the porous film is a woven fabric or a nonwoven fabric, the weight per unit area is preferably 5 to 30 g/m$^2$, more preferably 6 to 15g/m$^2$.

A suitable support is a laminated film of a polyester film (preferably, a polyethylene terephthalate film) having a thickness of 1.5 to 6 μm and a polyester (preferably, polyethylene terephthalate) nonwoven fabric having a weight per unit area of 6 to 15 g/m$^2$.

The release liner is not particularly limited as long as it is preliminarily subjected to release treatment and ensures sufficiently light releasability. Examples thereof include films made of polyester, polyvinyl chloride, polyvinylidene chloride, polyethylene terephthalate, or the like; paper such as wood-free paper and glassine paper; and laminate films of wood-free paper or glassine paper and polyolefin, which are preliminarily treated to be releasable by applying, for example, silicone resin or fluorine resin to the surface to be in contact with the adhesive layer.

The thickness of the release liner is preferably 10 to 200 μm, more preferably 25 to 100 μm.

The release liner is preferably formed from polyester (in particular, polyethylene terephthalate) resin in view of factors such as barrier properties and cost. In this case, the thickness of the release liner is preferably about 25 to 100 μm in view of handleability.

In administration of the vaccine pharmaceutical composition of the present invention to the subject, the therapeutically effective amount of the antigen may widely vary depending on the severity of the disease, age and relative health of the subject, and other known factors. Generally, satisfactory results can be obtained at a dose of about 0.1 μg/kg body weight to 1 g/kg body weight per day. The immunity induction promoter that is the ion channel drug is simultaneously or sequentially administered with the antigen. Simultaneous administration is preferred.

In administration of the composition for immunity induction promotion of the present invention and in administration of a vaccine pharmaceutical composition containing the composition for immunity induction promotion, the therapeutically effective amount of the immunity induction promoter that is the ion channel drug may widely vary depending on the specific type of the ion channel drug used, the presence or absence of other immunity induction promoter(s), and other factors. Generally, satisfactory results can be obtained at a dose of about 0.01 μg/kg body weight to 1 g/kg body weight per day.

The daily dose may be administered in one time, or may be split into multiple doses (i.e., two or more doses, for example, 2, 3, 4, or 5 doses). Preferably, the period of continuous administration per dose is appropriately determined in the range from 1 minute to 7 days. Preferably, the administration interval is appropriately selected from once a day to once a year (for example, once a day, once every 2 days, once every 3 days, once a week, once every 2 weeks, once a month, once every 3 months, once every 6 months, once a year, etc.), or longer administration intervals, depending on the condition of patients, severity of the disease, and whether it is for therapeutic purposes or preventive purposes. Generally, for the therapeutic purposes for a patient actually having a severe disease, the vaccine pharmaceutical composition of the present invention is preferably administered at a higher frequency and/or in a higher dose, while for the preventive purposes for patients not having a disease, the vaccine pharmaceutical composition of the present invention is preferably administered at a lower frequency and/or in a lower dose.

- Advantageous Effects of Invention

The vaccine pharmaceutical composition of the present invention containing a first immunity induction promoter that is an ion channel drug that affects an ion channel or an ion pump can change the intracellular ion concentration to effectively induce cellular immunity and/or humoral immunity.

Since the ion channel drugs according to the present invention include many drugs widely perceived to be highly safe, they are considered to cause less adverse effects.

The vaccine pharmaceutical composition of the present invention can be not only subcutaneously, intradermally, and intramuscularly injected, but also transdermally and mucosally administrated. The composition thus leads to excellent compliance and offers, for example, the following advantages: The composition can be non-invasively administered, allowing painless administration and freeing patients from fear for injections. As the composition is easy to administer, patients can administer it by themselves, which reduces the risk of infections of healthcare workers via needle stick injury, and also reduces the frequency of hospital visits when repetitive administrations are required, contributing to improved quality of life of patients. Moreover, medical wastes requiring specific waste treatment, such as injection needles, are not generated. The composition in a patch form such as a poultice or a tape is advantageous in that it allows secure administration of a predetermined dose and control of the drug releasing rate at any rate, and that it does not stick to unintended sites when administered. The composition in a patch form is advantageous also in that since a patch can easily be removed, patients can immediately stop the administration by themselves by removing the patch from the application site if any adverse effect occurs. The vaccine pharmaceutical composition of the present invention is also advantageous in that it has a significantly improved efficacy compared with an antigen administered alone. Moreover, the vaccine pharmaceutical composition of the present invention is advantageous in that transdermal administration and mucosal administration thereof can induce higher immunity than administration by injection.

BRIEF DESCRIPTION OF DRAWINGS

Fig. 1 is a graph showing the evaluation results of the number of IFN-γ-producing cell spots in mouse spleen cells after transdermal administration of a cream for transdermal administration obtained in each of Examples 1 to 14 and Comparative Examples 1 and 2.

Fig. 2 is a graph showing the evaluation results of the number of IFN-γ-producing cell spots in mouse spleen cells after transdermal administration of a tape for transdermal administration obtained in each of Examples 15 to 26 and Comparative Examples 3 to 8.

Fig. 3 is a graph showing the results of the OVA-specific IgG antibody titer in mouse serum after transnasal administration of a solution for transnasal administration obtained in each of Examples 27 to 34 and Comparative Example 9.

Fig. 4 is a graph showing the results of the OVA-specific IgG antibody titer in mouse serum after sublingual administration of a solution for sublingual administration obtained in each of Examples 35 to 42 and Comparative Example 10.

Fig. 5 is a graph showing the results of the OVA-specific IgG antibody titer in mouse serum after sublingual administration of a solid formulation for sublingual administration obtained in each of Examples 43 to 58 and Comparative Examples 11 and 12.

Fig. 6 is a graph showing the results of the OVA-specific IgG antibody titer in mouse serum after subcutaneous administration of a solution for subcutaneous administration obtained in each of Examples 59 to 63 and Comparative Example 13.

Fig. 7 is a graph showing the results of the OVA-specific IgG antibody titer in mouse serum after transdermal administration of a cream for transdermal administration obtained in each of Examples 64 to 68 and Comparative Example 14.

DESCRIPTION OF EMBODIMENTS

The present invention is specifically described with reference to, but not limited to, the following examples. Herein, "%" means "% by mass" and "part(s)" means "part(s) by mass" unless otherwise specified.

(Examples 1 to 14, Comparative Examples 1 and 2)

(Preparation of creams for transdermal administration)

Creams for transdermal administration having a composition as shown in Table 1 below were prepared.
Specifically, an antigen (5% by mass), a first immunity induction promoter (3% by mass), and optionally a second immunity induction promoter (1% by mass), and dimethylsulfoxide (DMSO) (15% by mass) mentioned below were mixed in amounts specified in Table 1. To the resulting mixture was added a base (base cream) to a total mass of 100% by mass, followed by mixing to give a cream for transdermal administration. The base cream used was prepared by mixing materials shown in Table 8 in amounts as specified. White petrolatum, sorbitan monostearate, isostearic acid, benzyl alcohol, stearyl alcohol, polysorbate 60, concentrated glycerin, and dimethylsulfoxide (DMSO) were purchased from Wako Pure Chemical Industries, Ltd. Cetanol was purchased from Tokyo Chemical Industry Co., Ltd.
A PET film/PET nonwoven fabric laminate (area: 0.7 cm$^2$) was attached to the center of an adhesive tape for fixing in such a manner that the PET film was in contact with the tape, thereby preparing a complex base. To a nonwoven fabric portion of the obtained complex base was applied 4 mg of the cream for transdermal administration, thereby preparing an administration sample in an immunity test.

(First immunity induction promoters)

Suramin sodium (Wako Pure Chemical Industries, Ltd.)
Amiloride hydrochloride (Sigma-Aldrich Co. LLC)
Amlodipine (Sigma-Aldrich Co. LLC)
Sodium valproate (Wako Pure Chemical Industries, Ltd.)
Nicorandil (Wako Pure Chemical Industries, Ltd.)

Minoxidil (LKT Laboratories, Inc.)
Niflumic acid (Sigma-Aldrich Co. LLC)
Triamterene (Sigma-Aldrich Co. LLC)
Manidipine (LKT Laboratories, Inc.)
Dofetilide (Sigma-Aldrich Co. LLC)
Glibenclamide (Wako Pure Chemical Industries, Ltd.)
Digitoxin (Wako Pure Chemical Industries, Ltd.)
Digoxin (Tokyo Chemical Industry Co., Ltd.)

Antigens (HER2/neu_E75 (HER2/neu_E75 peptide, cancer antigen peptide), IPEP87 (IPEP87 peptide, infectious pathogen-derived antigen peptide), MAGE-A3_A02 (MAGE3_A02 peptide, cancer antigen peptide) and PADRE as a second immunity induction promoter (helper T cell-activating peptide) were obtained by chemical synthesis and HPLC purification.

OVAp (SIGMA-ALDRICH) was used as a model antigen.

<Evaluation 1>

The creams for transdermal administration obtained in the examples and comparative examples were evaluated as follows.

(Evaluation on cellular immunity-inducing effect)

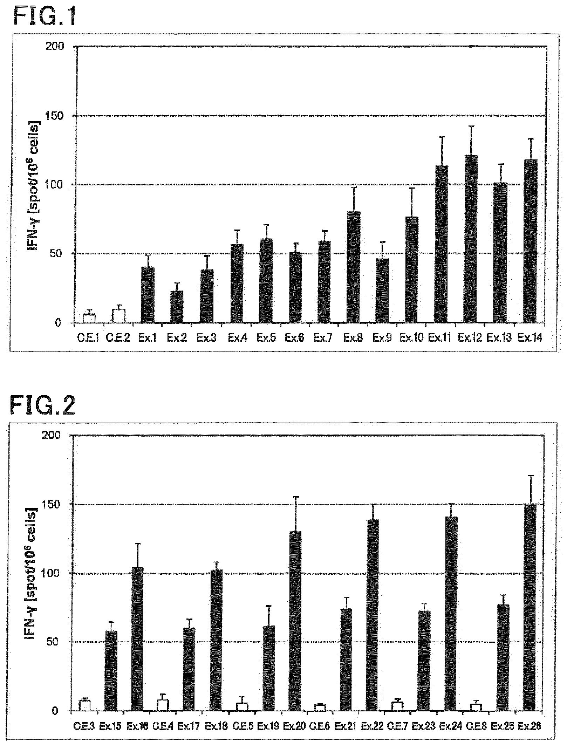

According to the procedure described below, the cream for transdermal administration was used to carry out a mouse immunity test using an animal model for immunological evaluation. Subsequently, the level of induction of antigen-specific cellular immunity was evaluated by ELISPOT assay. Fig. 1 shows the evaluation results.

(1) Animal model for immunological evaluation

The "animal model for immunological evaluation" herein refers to an animal model for evaluating the immunity-inducing properties of a vaccine pharmaceutical composition (in the present case, a cream for transdermal administration), and specifically refers to an animal model for evaluating the level of the cellular immunity induced by the cream for transdermal administration.

In consideration of the compatibility between the antigen in the cream for transdermal administration and MHC class I molecules of the animal, the animal model used for immunological evaluation was an animal with which induction of the cellular immunity by the antigen in the cream for transdermal administration can be evaluated.

(2) Mouse immunity test of creams for transdermal administration

According to Table 1, a mouse was provided and its back was shaved. After a certain rearing period for recovery from skin damage caused by the shaving, 4 mg of the cream for transdermal administration was administered to the skin of the back for 24 hours and then removed therefrom. The mouse was reared for six days. Six days after the administration, the spleen was extracted, and a spleen cell suspension was prepared. Spleen cells ($1 \times 10^6$ cells/well) and an antigen peptide (100 $\mu$M) together with a culture fluid were poured into wells of an ELISPOT plate on which an anti-mouse IFN-$\gamma$ antibody was immobilized, and co-cultured under the culture conditions of 37°C and 5% $CO_2$ for 20 hours. The number of IFN-$\gamma$-producing cell spots was evaluated by the ELISPOT assay. Table 1 shows the number of IFN-$\gamma$-producing cell spots as the "Immunity result".

[Table 1]

| No. | Administration route | Dosage form | Antigen | | First immunity induction promoter | | Second immunity induction promoter | | Immunity evaluation mouse | Immunity results |
|---|---|---|---|---|---|---|---|---|---|---|
| | | | Name | Amount [mass%] | Name | Amount [mass%] | Name | Amount [mass%] | | |
| Comparative Example 1 | Transdermal | Cream | OVAp | 5 | - | - | PADRE | - | C57BL/6 | 6.3 |
| Comparative Example 2 | Transdermal | Cream | OVAp | 5 | - | - | PADRE | 1 | C57BL/6 | 9.8 |
| Example 1 | Transdermal | Cream | OVAp | 5 | Suramin sodium | 3 | PADRE | - | C57BL/6 | 40.0 |
| Example 2 | Transdermal | Cream | OVAp | 5 | Amiloride hydrochloride | 3 | PADRE | - | C57BL/6 | 22.8 |
| Example 3 | Transdermal | Cream | OVAp | 5 | Amlodipine | 3 | PADRE | - | C57BL/6 | 38.0 |
| Example 4 | Transdermal | Cream | OVAp | 5 | Sodium valproate | 3 | PADRE | - | C57BL/6 | 56.5 |
| Example 5 | Transdermal | Cream | OVAp | 5 | Nicorandil | 3 | PADRE | - | C57BL/6 | 60.3 |
| Example 6 | Transdermal | Cream | OVAp | 5 | Minoxidil | 3 | PADRE | - | C57BL/6 | 50.4 |
| Example 7 | Transdermal | Cream | OVAp | 5 | Niflumic acid | 3 | PADRE | - | C57BL/6 | 58.8 |
| Example 8 | Transdermal | Cream | OVAp | 5 | Suramin sodium | 3 | PADRE | 1 | C57BL/6 | 80.5 |
| Example 9 | Transdermal | Cream | OVAp | 5 | Amiloride hydrochloride | 3 | PADRE | 1 | C57BL/6 | 46.0 |
| Example 10 | Transdermal | Cream | OVAp | 5 | Amlodipine | 3 | PADRE | 1 | C57BL/6 | 76.5 |
| Example 11 | Transdermal | Cream | OVAp | 5 | Sodium valproate | 3 | PADRE | 1 | C57BL/6 | 113.4 |
| Example 12 | Transdermal | Cream | OVAp | 5 | Nicorandil | 3 | PADRE | 1 | C57BL/6 | 120.9 |
| Example 13 | Transdermal | Cream | OVAp | 5 | Minoxidil | 3 | PADRE | 1 | C57BL/6 | 101.0 |
| Example 14 | Transdermal | Cream | OVAp | 5 | Niflumic acid | 3 | PADRE | 1 | C57BL/6 | 118.0 |

(Examples 15 to 26, Comparative Examples 3 to 8)

(Preparation of tapes for transdermal administration)

Tapes for transdermal administration having a composition as shown in Table 2 below were prepared. Specifically, an antigen, a first immunity induction promoter, and a second immunity induction promoter were mixed in amounts specified in Table 2. To the resulting mixture were added an adhesive base and an organic solvent (ethyl acetate) shown in Table 2 to a total mass of the components and the adhesive base after drying of the organic solvent of 100% by mass, followed by mixing to give an adhesive solution. The adhesive solution was spread on a release liner to a thickness after drying of about 80 $\mu$m, followed by drying to remove the organic solvent. Thus, an adhesive layer was formed. The release liner used was a polyethylene terephthalate (PET) liner (thickness: 75 $\mu$m) subjected to silicone release treatment. The obtained adhesive layer was attached to a support to give a tape for transdermal administration. The support used was a polyethylene terephthalate (PET) film (thickness: 25 $\mu$m).

The tape for transdermal administration was cut to have an area of 0.7 cm$^2$. This was used as an administration sample in an immunity test. The release liner was peeled upon administration.

<Evaluation 2>

The tapes for transdermal administration obtained in the examples and comparative examples were evaluated as follows.

(Evaluation on cellular immunity-inducing effect)

The level of induction of an antigen-specific cellular immunity was evaluated in the same manner as in the evaluation of the creams for transdermal administration. Fig. 2 shows the evaluation results.

[Table 2]

| No. | Administration route | Dosage form | Base | Antigen | | First immunity induction promoter | | Second immunity induction promoter | | Immunological evaluation mouse | Immunity result | |
|---|---|---|---|---|---|---|---|---|---|---|---|---|
| | | | | Name | Amount [mass%] | Name | Amount [mass%] | Name | Amount [mass%] | | Average | [cells/well] |
| Comparative Example 3 | Transdermal | Tape | Acrylic base | HER2/neu_E75 | 10 | - | - | PADRE | 1 | Genetically engineered | 7.3 | |
| Example 15 | Transdermal | Tape | Acrylic base | HER2/neu_E75 | 10 | Niflumic acid | 1 | - | 1 | Genetically engineered | 57.3 | $2\times10^6$ |
| Example 16 | Transdermal | Tape | Acrylic base | HER2/neu_E75 | 10 | Niflumic acid | 1 | PADRE | 1 | Genetically engineered | 104.0 | |
| Comparative Example 4 | Transdermal | Tape | PIB | HER2/neu_E75 | 10 | - | - | PADRE | 1 | Genetically engineered | 8.0 | |
| Example 17 | Transdermal | Tape | PIB | HER2/neu_E75 | 10 | Niflumic acid | 1 | - | 1 | Genetically engineered | 59.5 | $2\times10^6$ |
| Example 18 | Transdermal | Tape | PIB | HER2/neu_E75 | 10 | Niflumic acid | 1 | PADRE | 1 | Genetically engineered | 102.0 | |
| Comparative Example 5 | Transdermal | Tape | Acrylic base | IPEP87 | 10 | - | - | PADRE | 1 | Genetically engineered | 5.5 | |
| Example 19 | Transdermal | Tape | Acrylic base | IPEP87 | 10 | Niflumic acid | 1 | - | 1 | Genetically engineered | 61.0 | $2\times10^6$ |
| Example 20 | Transdermal | Tape | Acrylic base | IPEP87 | 10 | Niflumic acid | 1 | PADRE | 1 | Genetically engineered | 129.8 | |
| Comparative Example 6 | Transdermal | Tape | PIB | IPEP87 | 10 | - | - | PADRE | 1 | Genetically engineered | 4.3 | |
| Example 21 | Transdermal | Tape | PIB | IPEP87 | 10 | Niflumic acid | 1 | - | 1 | Genetically engineered | 73.8 | $2\times10^6$ |
| Example 22 | Transdermal | Tape | PIB | IPEP87 | 10 | Niflumic acid | 1 | PADRE | 1 | Genetically engineered | 138.5 | |
| Comparative Example 7 | Transdermal | Tape | Acrylic base | MAGE-A3_A02 | 10 | - | - | PADRE | 1 | Genetically engineered | 138.5 | |

(continued)

| No. | Administration route | Dosage form | Base | Antigen | | First immunity induction promoter | | Second immunity induction promoter | | Immunological evaluation mouse | Immunity result | |
|---|---|---|---|---|---|---|---|---|---|---|---|---|
| | | | | Name | Amount [mass%] | Name | Amount [mass%] | Name | Amount [mass%] | | Average | [cells/well] |
| Example 23 | Transdermal | Tape | Acrylic base | MAGE-A3_A02 | 10 | Niflumic acid | 1 | - | 1 | Genetically engineered | 72.3 | $2 \times 10^6$ |
| Example 24 | Transdermal | Tape | Acrylic base | MAGE-A3_A02 | 10 | Niflumic acid | 1 | PADRE | 1 | Genetically engineered | 140.5 | |
| Comparative Example 8 | Transdermal | Tape | PIB | MAGE-A3_A02 | 10 | - | - | PADRE | 1 | Genetically engineered | 4.8 | |
| Example 25 | Transdermal | Tape | PIB | MAGE-A3_A02 | 10 | Niflumic acid | 1 | - | 1 | Genetically engineered | 76.8 | $2 \times 10^6$ |
| Example 26 | Transdermal | Tape | PIB | MAGE-A3_A02 | 10 | Niflumic acid | 1 | PADRE | 1 | Genetically engineered | 149.0 | |

(Examples 27 to 42, Comparative Examples 9 and 10)

(Preparation of solutions for transmucosal administration)

Solutions for transmucosal administration (transnasal administration or sublingual administration) having a composition as shown in Table 3 or 4 below were prepared. Specifically, an antigen (ovalbumin (OVA)) and a first immunity induction promoter were mixed in amounts specified in Table 3 or 4. To the resulting mixture were added saline to a total amount of 10 μL in the case of transnasal administration or 30 pL in the case of sublingual administration, followed by mixing to give a solution for transmucosal administration (transnasal administration or sublingual administration).

(Examples 43 to 58, Comparative Examples 11 and 12)

(Preparation of solid formulations for sublingual administration)

Solid formulations (freeze-dried formulations or films) for sublingual administration having a composition as shown in Table 5 below were prepared. Specifically, an antigen (ovalbumin (OVA)), a first immunity induction promoter, and hydroxypropyl cellulose (HPC-SSL available from Nippon Soda Co., Ltd.) as a base were mixed in amounts specified in Table 5. To the resulting mixture was added saline, followed by mixing to give a formulation solution. Then, the formulation solution was dispensed in 25-mg portions and freeze-dried to give a freeze-dried formulation, or subjected to low-pressure drying to give a film. As the immunity induction promoter that is a nuclear receptor ligand, those used as the immunity induction promoters in preparation of solutions for transmucosal administration were used.

<Evaluation 3>

The solutions for transmucosal administration or solid formulations for sublingual administration obtained in the examples and comparative examples were evaluated as follows.

(Evaluation on humoral immunity-inducing effect)

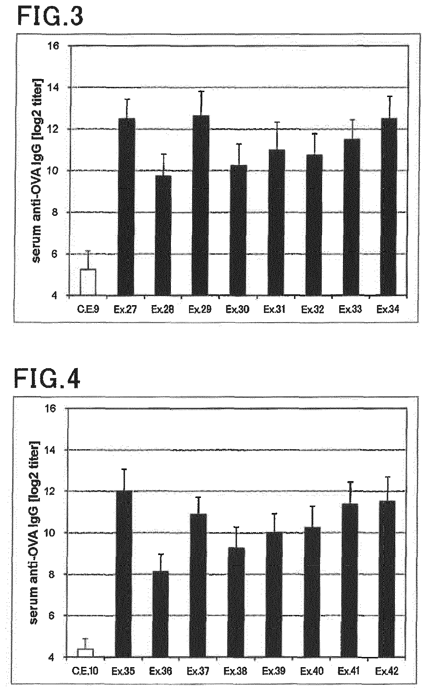

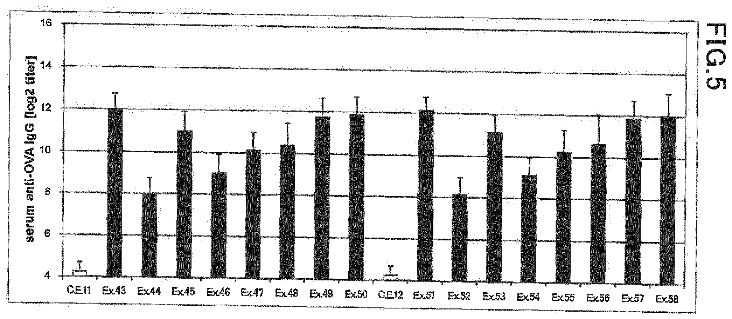

According to the procedure described below, the solution for transmucosal administration or the solid formulation for sublingual administration was used to carry out a mouse immunity test using an animal model for immunological evaluation. Subsequently, the systemic immune response was evaluated by measurement of the antigen (OVA)-specific IgG antibody in the mouse serum. Figs. 3 to 5 show the evaluation results.

(1) Mouse immunity test of solutions for transmucosal administration or solid formulations for sublingual administration

Preliminarily prepared mice (BALB/c mouse, 7-week-old female) were anesthetized. In the case of transnasal administration (Examples 27 to 34, Comparative Example 9), a solution for transmucosal administration in an amount of 10 μL was administered. In the case of sublingual administration (Examples 35 to 42, Comparative Example 10), a solution for transmucosal administration in an amount of 30 μL was administered. A solid formulation for sublingual administration (Examples 43 to 58, Comparative Examples 11 to 12) was administered. One week after the administration, each mouse was again anesthetized and subjected to the same administration. One week after the second administration, the mouse serum was collected.

(2) ELISA

(Method for determining antigen-specific IgG antibody titer in mouse serum (ELISA assay))

To each well of a 96-well plate for ELISA was added 100 μL of an OVA-containing solution (100 μg/mL) diluted with carbonate buffer, followed by standing overnight.
The wells were washed three times with preliminarily prepared wash (Tween 20-containing PBS), and to each well was added 200 μL of a blocking solution prepared by diluting a blocking agent (Block Ace available from Sumitomo Dainippon Pharma Co., Ltd.) in purified water to 4 g/100 mL. This was followed by standing for 2 hours at room temperature. The wells were then washed three times with wash.
The collected mouse serum was centrifuged at 4°C and 3000 g for 10 minutes, and the supernatant was recovered. The supernatant was diluted in two-fold increments using a solution prepared by diluting a blocking agent in a phosphate buffer (Nacalai Tesque, Inc.) to 0.4 g/100 mL. The diluted solution was added to wells (50 μL for each well),

followed by standing for 2 hours at room temperature.

The wells were then washed three times with wash. An HRP-labeled anti-mouse IgG antibody (Goat-anti mouse IgG Fc HRP, BETHYL) was diluted 10000-fold using a solution prepared by diluting a blocking agent in a phosphate buffer (Nacalai Tesque, Inc.) to 0.4 g/100 mL. To each well was added 100 $\mu$L of the resulting solution, followed by standing for 1 hour at room temperature.

The wells were then washed three times with wash, and 100 $\mu$L of a TMB solution (ELISA POD TMB kit available from Nacalai Tesque, Inc.) was added to each well, followed by standing for 30 minutes in a dark place.

Thereafer, 100 $\mu$L of a 1M sulfuric acid solution was added to each well, and the 96-well plate was subjected to measurement of absorbance at 450 nm with a microplate reader (SpectraMax M2e available from Molecular Devices, LLC.). The IgG antibody titer in the mouse serum was determined as Log2 titer based on the absorbance at the incremental dilution. Figs. 3 to 5 show the evaluation results.

[Table 3]

| No. | Administration route | Dosage form | Antigen | | First immunity induction promoter | | Affected channel or pump | Immunological evaluation mouse | IgG antibody titer [Log2 titer] |
|---|---|---|---|---|---|---|---|---|---|
| | | | Name | Amount [ug/mouse] | Name | Amount [ug/mouse] | | | |
| Comparative Example 9 | Transnasal | Solution | OVA | 1 | - | - | - | BALB/c | 5.3 |
| Example 27 | Transnasal | Solution | OVA | 1 | Suramin sodium | 20 | Na$^+$ channel | BALB/c | 12.5 |
| Example 28 | Transnasal | Solution | OVA | 1 | Triamterene | 100 | Na$^+$ channel | BALB/c | 9.8 |
| Example 29 | Transnasal | Solution | OVA | 1 | Amlodipine | 20 | Ca$^{2+}$ channel | BALB/c | 12.6 |
| Example 30 | Transnasal | Solution | OVA | 1 | Manidipine | 20 | Ca$^{2+}$ channel | BALB/c | 10.3 |
| Example 31 | Transnasal | Solution | OVA | 1 | Dofetilide | 20 | K$^+$ channel | BALB/c | 11.0 |
| Example 32 | Transnasal | Solution | OVA | 1 | Glibenclamide | 200 | K+ channel | BALB/c | 10.8 |
| Example 33 | Transnasal | Solution | OVA | 1 | Digitoxin | 20 | Na$^+$/K$^+$ pump | BALB/c | 11.5 |
| Example 34 | Transnasal | Solution | OVA | 1 | Digoxin | 100 | Na$^+$/K$^+$ pump | BALB/c | 12.5 |

[Table 4]

| No. | Administration route | Dosage form | Antigen | | First immunity induction promoter | | Affected channel or pump | Immunological evaluation mouse | IgG antibody titer [Log2 titer] |
|---|---|---|---|---|---|---|---|---|---|
| | | | Name | Amount [ug/mouse] | Name | Amount [ug/mouse] | | | |
| Comparative Example 10 | Sublingual | Solution | OVA | 10 | - | - | - | BALB/c | 4.4 |
| Example 35 | Sublingual | Solution | OVA | 10 | Suramin sodium | 50 | $Na^+$ channel | BALB/c | 12.0 |
| Example 36 | Sublingual | Solution | OVA | 10 | Triamterene | 100 | $Na^+$ channel | BALB/c | 8.1 |
| Example 37 | Sublingual | Solution | OVA | 10 | Amlodipine | 50 | $Ca^{2+}$ channel | BALB/c | 10.9 |
| Example 38 | Sublingual | Solution | OVA | 10 | Manidipine | 50 | $Ca^{2+}$ channel | BALB/c | 9.3 |
| Example 39 | Sublingual | Solution | OVA | 10 | Dofetilide | 50 | $K^+$ channel | BALB/c | 10.0 |
| Example 40 | Sublingual | Solution | OVA | 10 | Glibenclamide | 200 | $K^+$ channel | BALB/c | 10.3 |
| Example 41 | Sublingual | Solution | OVA | 10 | Digitoxin | 50 | $Na^+/K^+$ pump | BALB/c | 11.4 |
| Example 42 | Sublingual | Solution | OVA | 10 | Digoxin | 200 | $Na^+/K^+$ pump | BALB/c | 11.5 |

[Table 5]

Composition (parts by mass)

| Component name | | | Example | | | | | | | | | | | | | | | | Comparative Example | |
|---|---|---|---|---|---|---|---|---|---|---|---|---|---|---|---|---|---|---|---|---|---|
| | | | 43 | 44 | 45 | 46 | 47 | 48 | 49 | 50 | 51 | 52 | 53 | 54 | 55 | 56 | 57 | 58 | 11 | 12 |
| Antigen | OVA | | 1 | 1 | 1 | 1 | 1 | 1 | 1 | 1 | 1 | 1 | 1 | 1 | 1 | 1 | 1 | 1 | 1 | 1 |
| Immunostimulant | First immunity induction promoter | Suramin sodium | 10 | - | - | - | - | - | - | - | 10 | - | - | - | - | - | - | - | - | - |
| | | Triamterene | - | 10 | - | - | - | - | - | - | - | 10 | - | - | - | - | - | - | - | - |
| | | Amlodipine | - | - | 10 | - | - | - | - | - | - | - | 10 | - | - | - | - | - | - | - |
| | | Manidipine | - | - | - | 10 | - | - | - | - | - | - | - | 10 | - | - | - | - | - | - |
| | | Dofetilide | - | - | - | - | 10 | - | - | - | - | - | - | - | 10 | - | - | - | - | - |
| | | Glibenclamide | - | - | - | - | - | 10 | - | - | - | - | - | - | - | 10 | - | - | - | - |
| | | Digitoxin | - | - | - | - | - | - | 10 | - | - | - | - | - | - | - | 10 | - | - | - |
| | | Digoxin | - | - | - | - | - | - | - | 10 | - | - | - | - | - | - | - | 10 | - | - |
| Base | HPC-SSL | | 250 | 250 | 250 | 250 | 250 | 250 | 250 | 250 | 250 | 250 | 250 | 250 | 250 | 250 | 250 | 250 | 250 | 250 |
| | Saline | | 739.0 | 739.0 | 739.0 | 739.0 | 739.0 | 739.0 | 739.0 | 739.0 | 739.0 | 739.0 | 739.0 | 739.0 | 739.0 | 739.0 | 739.0 | 739.0 | 749.0 | 749.0 |
| Dispensing amount [mg/mouse] | | | 25.0 | 25.0 | 25.0 | 25.0 | 25.0 | 25.0 | 25.0 | 25.0 | 25.0 | 25.0 | 25.0 | 25.0 | 25.0 | 25.0 | 25.0 | 25.0 | 25.0 | 25.0 |
| IgG antibody titer [Log2 titer] | | | 12.0 | 8.0 | 11.0 | 9.0 | 10.1 | 10.4 | 11.8 | 11.9 | 12.1 | 8.1 | 11.1 | 9.1 | 10.3 | 10.6 | 11.9 | 12.0 | 4.3 | 4.3 |
| Dosage form | | | Solid (freeze-dried) | | | | | | | | Film | | | | | | | | Solid (freeze-dried) | Film |
| Administration route | | | Sublingual administration | | | | | | | | | | | | | | | | | |

(Examples 59 to 63, Comparative Example 13)

(Preparation of solutions for subcutaneous administration)

Formulations for subcutaneous administration having a composition as shown in Table 6 below were prepared. Specifically, an antigen (ovalbumin (OVA)) and a first immunity induction promoter were mixed in amounts specified in Table 6. To the resulting mixture was added saline to a total amount of 200 $\mu$L, followed by mixing to give a solution for subcutaneous administration.

<Evaluation 4>

The formulations for subcutaneous administration obtained in the examples and comparative example were evaluated as follows.

(Evaluation on humoral immunity-inducing effect)

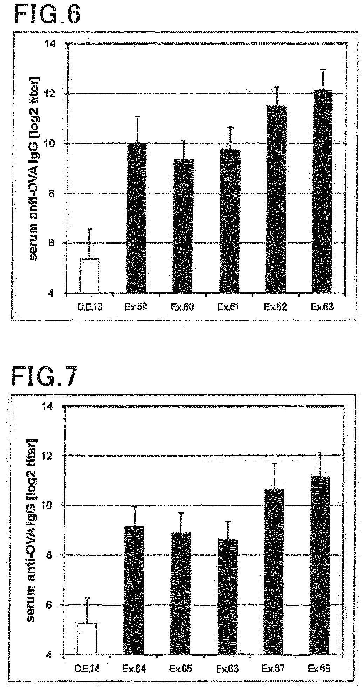

According to the procedure described below, the formulation for subcutaneous administration was used to carry out a mouse immunity test using an animal model for immunological evaluation. Subsequently, the systemic immune response was evaluated by measurement of the antigen (OVA)-specific IgG antibody in the mouse serum. Fig. 6 shows the evaluation results.

(1) Mouse immunity test of formulations for subcutaneous administration

Preliminarily prepared mice (BALB/c mouse, 7-week-old female) were anesthetized. To each mouse, 200 $\mu$L of the formulation was administered subcutaneously to the back of the mouse. One week after the administration, each mouse was again anesthetized and subjected to the same administration. One week after the second administration, the mouse serum was collected.

(2) ELISA

The antigen (OVA) -specific IgG antibody titer in the mouse serum was determined by ELISA in the same procedure as in <Evaluation 3>.

[Table 6]

| No. | Administration route | Dosage form | Antigen | | First immunity induction promoter | | Affected channel or pump | Immunological evaluation mouse | IgG antibody titer [Log2 titer] |
|---|---|---|---|---|---|---|---|---|---|
| | | | Name | Amount [ug] | Name | Amount [ug] | | | |
| Comparative Example 13 | Subcutaneous | Solution | OVA | 0.05 | - | - | - | BALB/c | 5.4 |
| Example 59 | Subcutaneous | Solution | OVA | 0.05 | Amiloride hydrochloride | 200 | Na$^+$ channel | BALB/c | 10.0 |
| Example 60 | Subcutaneous | Solution | OVA | 0.05 | Manidipine | 200 | Ca$^{2+}$ channel | BALB/c | 9.4 |
| Example 61 | Subcutaneous | Solution | OVA | 0.05 | Glibenclamide | 200 | K$^+$ channel | BALB/c | 9.8 |
| Example 62 | Subcutaneous | Solution | OVA | 0.05 | Digitoxin | 200 | Na$^+$/K$^+$ pump | BALB/c | 11.5 |
| Example 63 | Subcutaneous | Solution | OVA | 0.05 | Digoxin | 200 | Na$^+$/K$^+$ pump | BALB/c | 12.1 |

(Examples 64 to 68, Comparative Example 14)

(Preparation of creams for transdermal administration)

Creams for transdermal administration having a composition as shown in Table 7 below were prepared. Specifically, an antigen (ovalbumin (OVA)) and a first immunity induction promoter were mixed in amounts specified in Table 7. To the resulting mixture was added a base (base cream) to a total amount of 100 parts by mass, followed by mixing to give a cream for transdermal administration. The base cream used was prepared by blending and mixing the materials shown in Table 8 in amounts as specified.

White petrolatum, sorbitan monostearate, isostearic acid, benzyl alcohol, stearyl alcohol, polysorbate 60, and concentrated glycerin were purchased from Wako Pure Chemical Industries, Ltd. Cetanol was purchased from Tokyo Chemical Industry Co., Ltd.

A PET film/PET nonwoven fabric laminate (area: 0.7 cm$^2$) was attached to the center of an adhesive tape for fixing in such a manner that the PET film was in contact with the tape, thereby preparing a complex base. To a nonwoven fabric portion of the obtained complex base was applied 4 mg of the cream for transdermal administration, thereby preparing an administration sample for a mouse immunity test.

<Evaluation 5>

The creams for transdermal administration obtained in the examples and comparative example were evaluated as follows.

(Evaluation on humoral immunity-inducing effect)

According to the procedure described below, the cream for transdermal administration was used to carry out a mouse immunity test using an animal model for immunological evaluation. Subsequently, the systemic immune response was evaluated by measurement of the antigen (OVA)-specific IgG antibody titer in the mouse serum. Fig. 7 shows the evaluation results.

(1) Mouse immunity test of creams for transdermal administration

The right back of a mouse (C57BL/6 NCr mouse, 7-week-old female) was shaved in advance. After a rearing period for recovery from the skin damage caused by the shaving, 4 mg of the cream for transdermal administration was administered to the skin of the right back of the mouse, and the left back was shaved at the same time. Twenty-four hours later, the cream for transdermal administration on the right back was removed. One week after the administration, the cream for transdermal administration was similarly administered to the skin of the left back of the mouse, and removed 24 hours later. One week after the second administration, the mouse serum was collected.

(2) ELISA

The antigen (OVA)-specific IgG antibody titer in the mouse serum was determined by ELISA in the same procedure as in <Evaluation 3>.

[Table 7]

| No. | Administration route | Dosage form | Antigen | | First immunity induction promoter | | Affected channel or pump | Immunological evaluation mouse | IgG antibody titer [Log2 titer] |
|---|---|---|---|---|---|---|---|---|---|
| | | | Name | Amount [parts by mass] | Name | Amount [parts by mass] | | | |
| Comparative Example 14 | Transdermal | Cream | OVA | 5 | - | - | - | C57BL/6 | 5.3 |
| Example 64 | Transdermal | Cream | OVA | 5 | Amiloride hydrochloride | 5 | $Na^+$ channel | C57BL/6 | 9.1 |
| Example 65 | Transdermal | Cream | OVA | 5 | Manidipine | 5 | $Ca^{2+}$ channel | C57BL/6 | 8.9 |
| Example 66 | Transdermal | Cream | OVA | 5 | Glibenclamide | 5 | $K^+$ channel | C57BL/6 | 8.6 |
| Example 67 | Transdermal | Cream | OVA | 5 | Digitoxin | 5 | $Na^+/K^+$ pump | C57BL/6 | 10.6 |
| Example 68 | Transdermal | Cream | OVA | 5 | Digoxin | 5 | $Na^+/K^+$ pump | C57BL/6 | 11.1 |

[Table 8]

| Additive | Amount [parts by weight] |
|---|---|
| White petrolatum | 60.7 |
| Sorbitan monostearate | 0.7 |
| Isostearic acid | 12 |
| Benzyl alcohol | 2.4 |
| Cetanol | 2.4 |
| Stearyl alcohol | 3.5 |
| Polysorbate 60 | 3.5 |
| Concentrated glycerin | 2.4 |
| Purified water | 12.4 |
| Total | 100 |

INDUSTRIAL APPLICABILITY

The composition for immunity induction promotion and the vaccine pharmaceutical composition of the present invention are universally usable for induction of cellular immunity and/or humoral immunity against various antigens and can exert a high effect of inducing cellular immunity and/or humoral immunity.

## Claims

1. A composition for immunity induction promotion, comprising a first immunity induction promoter, the first immunity induction promoter being an ion channel drug that affects an ion channel or an ion pump.

2. The composition for immunity induction promotion according to claim 1, wherein the ion channel drug is at least one selected from the group consisting of a sodium channel agonist, a calcium channel agonist, a potassium channel agonist, a chloride channel agonist, and a sodium/potassium pump agonist.

3. A vaccine pharmaceutical composition comprising:

    an antigen for immunity induction; and
    the composition for immunity induction promotion according to claim 1 or 2.

**4.** The vaccine pharmaceutical composition according to claim 3 further comprising a second immunity induction promoter,
wherein the second immunity induction promoter is at least one immunostimulant selected from the group consisting of a Toll-like receptor (TLR) ligand, a cyclic dinucleotide, and a helper peptide.

**5.** The vaccine pharmaceutical composition according to claim 3 or 4,
which is administered to a body surface.

**6.** The vaccine pharmaceutical composition according to claim 3 or 4,
which is administered by intradermal injection, subcutaneous injection, or intramuscular injection.

# FIG.1

# FIG.2

## FIG.3

## FIG.4

# FIG.5

FIG.6

FIG.7

## INTERNATIONAL SEARCH REPORT

| International application No. |
| --- |
| PCT/JP2017/003631 |

A. CLASSIFICATION OF SUBJECT MATTER
*A61K39/39(2006.01)i, A61K39/00(2006.01)i*

According to International Patent Classification (IPC) or to both national classification and IPC

B. FIELDS SEARCHED

Minimum documentation searched (classification system followed by classification symbols)
A61K39/39, A61K39/00

Documentation searched other than minimum documentation to the extent that such documents are included in the fields searched
Jitsuyo Shinan Koho        1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
| --- | --- | --- |
| X | WO 2013/44419 A1 (BEIJING ADVACCINE BIOTECHNOLOGY CO., LTD.), 04 April 2013 (04.04.2013), claims; page 37, line 1 to page 38, line 26; examples 1 to 6 & US 2015/0044244 A1 | 1-6 |
| X | US 3523155 A (Henry E. Meunler), 04 August 1970 (04.08.1970), claims; examples (Family: none) | 1-6 |
| X | US 2005/0226894 A1 (Erika Jensen-Jarolim), 13 October 2005 (13.10.2005), claims; examples & WO 2003/059380 A2   & EP 1327450 A1 | 1,3,4 |

☒ Further documents are listed in the continuation of Box C.        ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 March 2017 (01.03.17) | 14 March 2017 (14.03.17) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

International application No.

PCT/JP2017/003631

C (Continuation).   DOCUMENTS CONSIDERED TO BE RELEVANT

| Category* | Citation of document, with indication, where appropriate, of the relevant passages | Relevant to claim No. |
|---|---|---|
| X | EP 2548555 A1 (Fondazione IRCCS Istituto Nazionale dei Tumori), 23 January 2013 (23.01.2013), claims; examples (Family: none) | 1,3-6 |
| X | Yasuaki AOKI, "Procainamide no Vaccine Koka Zokyo Sayo", Orthopaedic research science, 1986, vol.13, pages 718 to 721, particularly, fig. 1 to 2, tables 1 to 2, Study | 1-6 |
| X | Mai NISHIKAWA, "Ambroxol suppresses influenza virus multiplication in the airway by increasing antiviral factor levels", Shikoku Acta Medica, 2002, vol.58, no.3, pages 162 to 167, particularly, pages 164 to 165 | 1-6 |
| X | JP 2003-518076 A (The Trustees of the University of Pennsylvania), 03 June 2003 (03.06.2003), claims; paragraphs [0061] to [0065], [0072] to [0073]; examples & US 2008/0219953 A1 claims; paragraphs [0064] to [0079], [0085]; examples & WO 2001/045749 A1      & EP 1257298 A1 | 1-6 |
| X | Maria de Lourdes Mora-Garcia et al, Up-regulation of HLA class-I antigen expression and antigen-specific CTL response in cervical cancer cells by the demethylating agent hydralazine and the histone deacetylase inhibitor valproic acid, Jounal of Translational Medicine, 2006, Vol.4, pp.55, particularly, Abstract, Fig 1,5, Conclusion | 1-6 |
| X | Shuang Geng et al, Amiloride Enhances Antigen Specific CTL by Faciliting HBV DNA Vaccine Entry into Cells, PloS one, 2012, Vol.7, No.3, pp.e.33015, particularly, Abstract, Fig 1-5 | 1-6 |
| X | Micael Denkinger et al, Suramin has adjuvant properties and promotes expansion of antigen-specific Th1 and Th2 cells in vivo, International Immunopharmacology, 2004, Vol.4, No.1, pp.15-24, particularly, Abstract, Fig 1,3, 4, Discussion | 1-6 |
| P,X | JP 2016-34924 A (Hisamitsu Pharmaceutical Co., Inc.), 17 March 2016 (17.03.2016), claims; examples (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080193487 A
- JP 2002531415 T
- US 20080112974 A
- JP 2014169281 A

**Non-patent literature cited in the description**

- **HOSOI AKIHIRO et al.** *CANCER RESEARCH,* 2008, vol. 68, 3941-3949
- **ZHENGRONG CUI et al.** *PHARMACEUTICAL RESEARCH,* 2002, vol. 19 (7), 947-953
- Japanese Pharmacopoeia

SEQUENCE LISTING

<110> NITTO DENKO CORPORATION

<120> COMPOSITION FOR IMMUNITY INDUCTION PROMOTION AND VACCINE
PHARMACEUTICAL
       COMPOSITION

<130> N 3853EU - py

<140> EP 17 747 468.1
<141> 01.02.2017

<150> JP 2016-018278
<151> 02.02.2016

<160> 12

<170> PatentIn version 3.5

<210> 1
<211> 9
<212> PRT
<213> Homo sapiens

<400> 1

Ala Tyr Ala Cys Asn Thr Ser Thr Leu
1               5

<210> 2
<211> 9
<212> PRT
<213> Homo sapiens

<400> 2

Glu Tyr Ile Leu Ser Leu Glu Glu Leu
1               5

<210> 3
<211> 9
<212> PRT
<213> Homo sapiens

<400> 3

Thr Tyr Leu Pro Thr Asn Ala Ser Leu
1               5

<210> 4
<211> 9
<212> PRT
<213> Homo sapiens

<400> 4

Ile Met Pro Lys Ala Gly Leu Leu Ile
1               5

```
<210>  5
<211>  9
<212>  PRT
<213>  Hepatitis C virus

<400>  5

Asp Leu Met Gly Tyr Ile Pro Ala Val
1                   5


<210>  6
<211>  9
<212>  PRT
<213>  Homo sapiens

<400>  6

Val Leu Gln Glu Leu Asn Val Thr Val
1                   5


<210>  7
<211>  9
<212>  PRT
<213>  Homo sapiens

<400>  7

Lys Val Phe Gly Ser Leu Ala Phe Val
1                   5


<210>  8
<211>  9
<212>  PRT
<213>  Homo sapiens

<400>  8

Lys Val Ala Glu Ile Val His Phe Leu
1                   5


<210>  9
<211>  9
<212>  PRT
<213>  Hepatitis B virus

<400>  9

Trp Leu Ser Leu Leu Val Pro Phe Val
1                   5


<210>  10
<211>  9
<212>  PRT
<213>  Homo sapiens

<400>  10
```

```
Lys Ile Phe Gly Ser Leu Ala Phe Leu
1               5
```

```
<210>  11
<211>  9
<212>  PRT
<213>  Homo sapiens

<400>  11
```

```
Ser Thr Ala Pro Pro Val His Asn Val
1               5
```

```
<210>  12
<211>  13
<212>  PRT
<213>  Artificial Sequence

<220>
<223>  PADRE


<220>
<221>  MOD_RES
<222>  (1)..(1)
<223>  D-alanine

<220>
<221>  MOD_RES
<222>  (3)..(3)
<223>  cyclohexylalanine

<220>
<221>  MOD_RES
<222>  (13)..(13)
<223>  D-alanine

<400>  12
```

```
Ala Lys Ala Val Ala Ala Trp Thr Leu Lys Ala Ala Ala
1               5                   10
```